# EUROPÄISCHE PATENTSCHRIFT

(11) **EP 0 912 547 B1**
(45) Veröffentlichungstag und Bekanntmachung des Hinweises auf die Patenterteilung: **12.10.2005**
(21) Anmeldenummer: 97921808.8
(22) Anmeldetag: 28.04.1997
(51) Int. Cl.: C07D 401/04, A01N 43/40, C07D 405/04, C07D 409/04, C07D 417/04, C07F 9/6558, C07D 213/55, C07D 213/56, C07D 213/57

(54) **NEUE SUBSTITUIERTE PYRIDYLKETOENOLE**
NEW SUBSTITUTED PYRIDYL KETO ENOLS
NOUVEAUX PYRIDYLCETOENOLS SUBSTITUES

(30) Priorität: 10.05.1996 DE 19618831; 13.12.1996 DE 19651841
(43) Veröffentlichungstag der Anmeldung: 06.05.1999
(73) Patentinhaber: Bayer CropScience AG, 40789 Monheim (DE)
(72) Erfinder: LIEB, Folker, D-51375 Leverkusen (DE); HAGEMANN, Hermann, D-51375 Leverkusen (DE); WIDDIG, Arno, D-51519 Odenthal (DE); RUTHER, Michael, D-40789 Monheim (DE); FISCHER, Reiner, D-40789 Monheim (DE); BRETSCHNEIDER, Thomas, D-53797 Lohmar (DE); ERDELEN, Christoph, D-42799 Leichlingen (DE); WACHENDORFF-NEUMANN, Ulrike, D-56566 Neuwied (DE); GRAFF, Alan, D-51061 Köln (DE); DAHMEN, Peter, D-41470 Neuss (DE); DOLLINGER, Markus, D-51381 Leverkusen (DE); GALLENKAMP, Bernd, D-42113 Wuppertal (DE)
(86) Internationale Anmeldenummer: PCT/EP1997/002183
(87) Internationale Veröffentlichungsnummer: WO 1997/043275

(56) Entgegenhaltungen:
- EP-A- 0 230 266
- EP-A- 0 521 334
- EP-A- 0 521 827
- WO-A-92/16510
- US-A- 4 678 501
- US-A- 5 262 383
- HENNING H G ET AL: "Alternative routes to the synthesis of 2-furanoyl- and 2-pyrrolonyl-substituted quinolines" PHARMAZIE (PHARAT,00317144);88; VOL.43 (1); PP.45, HUMBOLDT-UNIV.;SEKT. CHEM.; BERLIN; GER. DEM. REP. (DD), XP002039515
- MEHNERT J ET AL: "Reactions of N-alkoxycyclimonium salts. XVI. Reactions of N-methoxyquinolinium compounds with C- and ambident C-N-nucleophiles" ARCH. PHARM. (WEINHEIM, GER.) (ARPMAS,03656233);88; VOL.321 (12); PP.897-901, UNIV. KIEL;PHARM. INST.; KIEL; D-2300; FED. REP. GER. (DE), XP002039516
- CHEMICAL ABSTRACTS, vol. 86, no. 1, 3.Januar 1977 Columbus, Ohio, US; abstract no. 715n, PILOTTI ET AL.: "Studies on the identification of tobacco ..." XP002039517
- CHEMICAL ABSTRACTS, vol. 109, no. 15, 10.Oktober 1988 Columbus, Ohio, US; abstract no. 128726c, OKAMOTO ET AL.: "A supported epoxidation catalyst for nucleophilic olefins" XP002039518

## Beschreibung

Die Erfindung betrifft neue pyridylsubstituierte cyclische Ketoenole, mehrere Verfahren und Zwischenprodukte zu ihrer Herstellung und ihre Verwendung als Schädlingsbekämpfungsmittel und Herbizide.

Bisher wurden keine pyridylsubstituiecten heterocyclischen Ketoenole beschrieben. Bekannt ist lediglich, daß bestimmte phenylsubstituierte cyclische Ketoenole als Insektizide, Akarizide und/oder Herbizide wirksam sind.

Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, DE 44 40 594, WO 94/01 997, WO 95/01 358, WO 95/20 572, EP-A-668 267 und WO 95/26 954).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Weiterhin sind 3-Aryl-Δ³-dihydrofuranon-Derivate mit herbiziden, akariziden und insektiziden Eigenschaften aus EP-A-528 156 und EP-A-0 647 637 bekannt. Auch 3-Aryl-Δ³-dihydrothiophen-on-Derivate sind bekannt (WO 95/26 345).

Im Phenylring substituierte Phenyl-pyron-Derivate mit herbiziden, akariziden und insektiziden Eigenschaften sind in EP-A-588 137 beschrieben.

Im Phenylring substituierte 5-Phenyl-1,3-thiazin-Derivate mit herbizider, akarizider und insektizider Wirkung sind in WO 94/ 14 785 beschrieben.

Die herbizide, akarizide und insektizide Wirksamkeit und/oder Wirkungsbreite, und die Pflanzenverträglichkeit dieser Verbindungen, insbesondere gegenüber Kulturpflanzen, ist jedoch nicht immer ausreichend.

Es wurden nun neue Verbindungen der Formel (I) 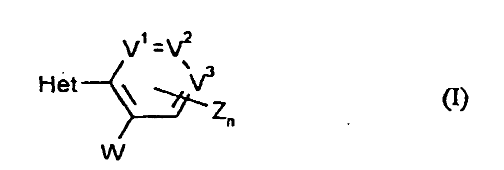 gefunden,
in welcher
A) V¹ für Stickstoff steht und
   V² für CH oder C-Z steht und
   V³ für CY steht oder
B) V¹ für CX steht und
   V² für Stickstoff steht und
   V³ für CY steht oder
C) V¹ für CX steht und
   V² für CH oder C-Z steht und
   V³ für Stickstoff steht
und in welcher
- W: für Wasserstoff, Nitro, Cyano, Halogen, C₁-C₆-Alkyl, C₂-C₆-Alkenyl, C₂-C₆-Alkinyl, C₁-C₆-Alkoxy, C₁-C₆-Alkylthio, C₁-C₄-Halogenalkyl, C₁-C₄-Halogenalkoxy oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht.
- X: für Wasserstoff, Halogen, C₁-C₆-Alkyl, C₂-C₆-Alkenyl, C₂-C₆-Alkinyl, C₁-C₆-Alkoxy, C₁-C₆-Alkylthio, C₁-C₄-Halogenalkyl, C₁-C₄-Halogenalkoxy, Cyano, Nitro oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht.
- Y: für Wasserstoff, Halogen, C₁-C₆-Alkyl, C₁-C₆-Alkoxy, C₁-C₆-Alkylthio, C₁-C₄-Halogenalkyl, C₁-C₄-Halogenalkoxy, Cyano oder Nitro steht.
- Z: für Halogen, C₁-C₆-Alkyl, C₁-C₆-Alkoxy, C₁-C₆-Alkylthio, C₁-C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht oder
- Y und Z: gemeinsam für jeweils gegebenenfalls durch Halogen, C₁-C₆-Alkyl, C₁-C₆-Alkoxy oder C₁-C₄-Halogenalkyl substituiertes C₃-C₄-Alkandiyl, C₃-C₄-Alkendlyl oder C₄-Alkandiendiyl stehen, in welchen gegebenenfalls ein bis drei Glieder durch Sauerstoff, Schwefel, Stickstoff oder eine Carbonylgruppe unabhängig voneinander ersetzt sein können, wobei in diesem Fall n für 1 steht, oder
- W und Z: gemeinsam für jeweils gegebenenfalls durch Halogen, C₁-C₆-Alkyl, C₁-C₆-Alkoxy oder C₁-C₄-Halogenalkyl substituiertes C₃-C₄-Alkandiyl, C₃-C₄-Alkendiyl oder C₄-Alkandiendiyl stehen, in welchen gegebenenfalls ein bis drei Glieder durch Sauerstoff, Schwefel, Stickstoff oder eine Carbonylgruppe unabhängig voneinander ersetzt sein können, wobei in diesem Fall n für 1 steht.
- n: für 0, 1 oder 2, steht wobei für n = 2 die Substituenten Z gleich oder verschieden sein können.
- Het: für die Gruppe 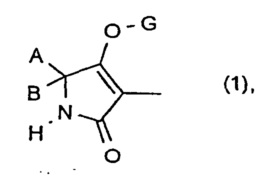 steht
- A: für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff steht.
- B: für Wasserstoff, C₁-C₁₂-Alkyl oder C₁-C₈-Alkoxy-C₁-C₆-alkyl steht oder
- A, B: und das Kohlenstoffatom an das sie gebunden sind, für C₃-C₁₀-Cycloalkyl oder C₅-C₁₀-Cycloalkenyl stehen, worin jeweils gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, für C₅-C₆-Cycloalkyl stehen, welches durch eine gegebenenfalls ein oder zwei Sauerstoff- und/oder Schwefelatome enthaltende Alkylendiyl-, oder durch eine Alkylendioxy- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind für C₃-C₈-Cycloalkyl oder C₅-C₈-Cycloalkenyl stehen, worin zwei Kohlenstoffatome durch jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl miteinander verbunden sind, worin jeweils gegebenenfalls eine Methylengruppe durch Sauerstoff oder Schwefel ersetzt ist.
- G: für Wasserstoff (a) oder für eine der Gruppen 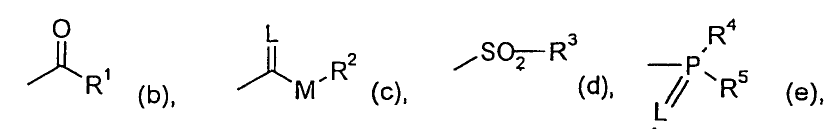 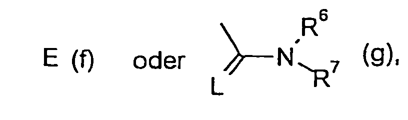 steht
in welchen
E für ein Metallionäquivalent oder ein Ammoniumion steht,
L für Sauerstoff oder Schwefel steht und
M für Sauerstoff oder Schwefel steht.
- R¹: für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht mit ein oder zwei Heteroatomen aus der Reihe Sauerstoff, Schwefel und Stickstoff.
- R²: für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht.
- R³: für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht.
- R⁴ und R⁵: unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen.
- R⁶ und R⁷: unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Je nachdem, ob V¹, V² oder V³ für Stickstoff steht, handelt es sich bei den Verbindungen der Formel (I) um Verbindungen der Formeln (I-A), (I-B) oder (I-C): 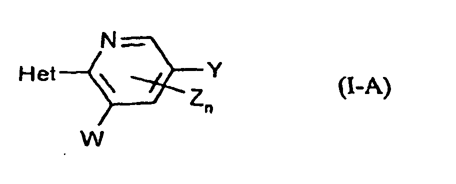 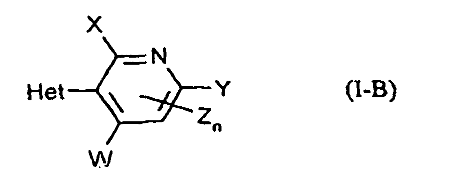 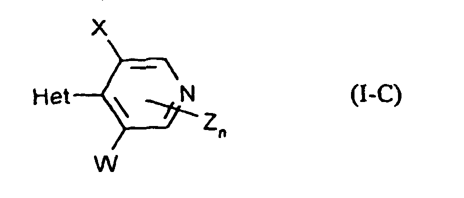 in welchen
Het, X, Y, Z, W und n die oben angegebene Bedeutung haben.

Zur Unterscheidung, in welcher Position des Pyridylrings sich der Stickstoff befindet, werden im folgenden in Bezeichnungen von Formeln auch von Vor- oder Zwischenprodukten bisweilen die Buchstaben (A), (B) oder (C) verwendet.

Unter Einbeziehung der Bedeutung (1) der Gruppe Het ergibt sich folgende hauptsächliche Struktur (I-1) : 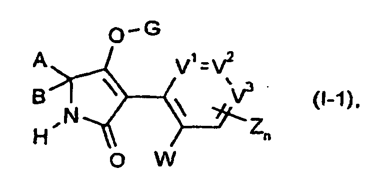 worin
A, B, D, G, W, V¹, V², V³, Z und n die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 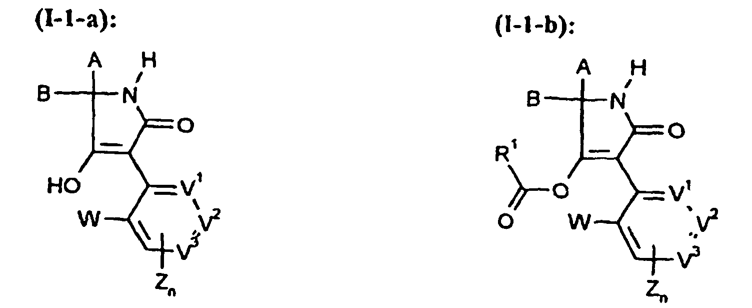 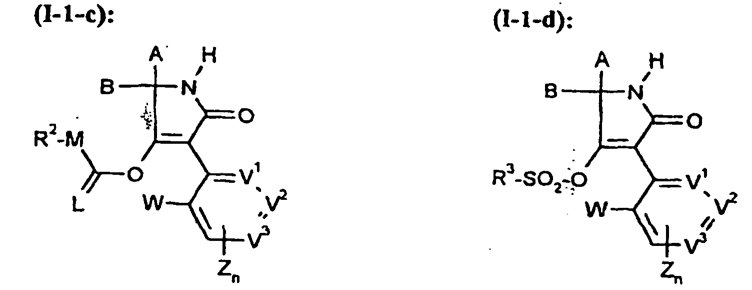 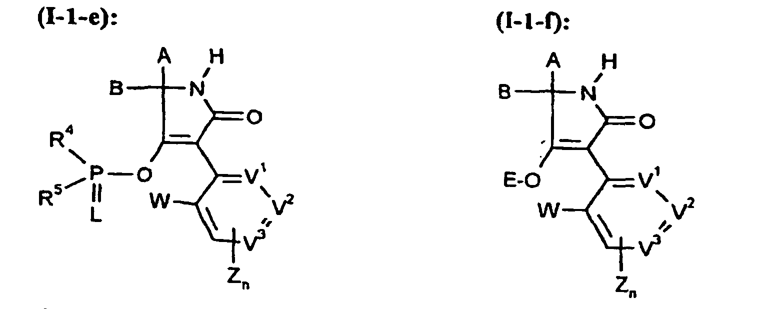 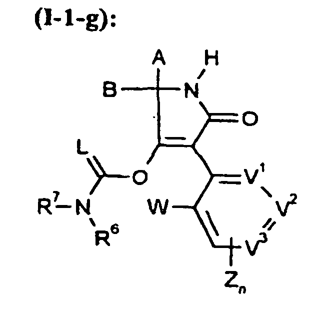 worin
A, B, E, L, M, W, V¹, V², V³, n, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach den im folgenden beschriebenen Verfahren erhält:
(A) Man erhält Verbindungen der Formel (I-1-a) 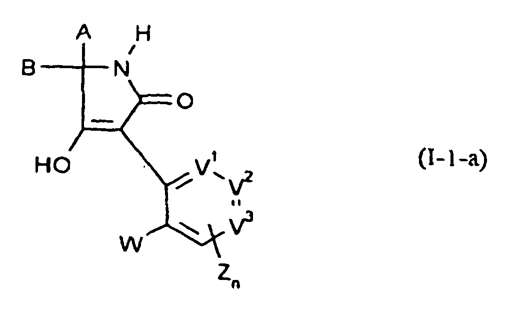 in welcher
   A, B, W, V¹, V², V³, n und Z die oben angegebenen Bedeutungen haben,
   wenn man
   Verbindungen der Formel (II) 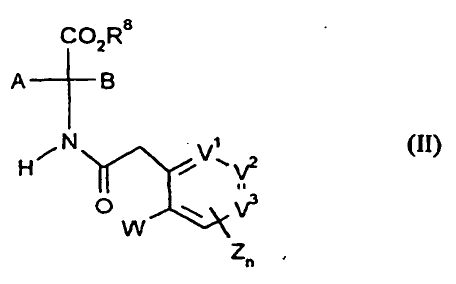 in welcher
   A, B, W, V¹, V², V³, n und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
   Außerdem wurde gefunden,
(F) daß man die Verbindungen der oben gezeigten Formel (I-1-b) in welcher A, B, R¹, W, V¹, V², V³, n und Z die oben angebenen Bedeutungen haben, erhält, wenn man Verbindungen der oben gezeigten Formel (I-1-a), in welcher A, B, W, V¹, V², V³, n und Z die oben angegebenen Bedeutungen haben,
   α) mit Säurehalogeniden der Formel (VIII) 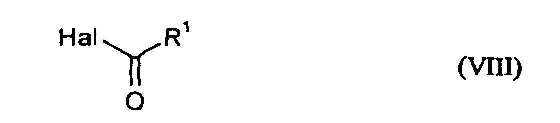 in welcher
      - R¹: die oben angegebene Bedeutung hat und
      - Hal: für Halogen (insbesondere Chlor oder Brom) steht
      oder
   β) mit Carbonsäureanhydriden der Formel (IX)

      R¹-CO-O-CO-R¹ (IX)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(G) daß man die Verbindungen der oben gezeigten Formel (I-1-c), in welcher A, B, R², W, M, V¹, V², V³, n und Z die oben angegebenen Bedeutungen haben und L für Sauerstoff steht, erhält, wenn man Verbindungen der oben gezeigten Formel (I-1-a), in welcher A, B, W, V¹, V², V³, n und Z die oben angegebenen Bedeutungen haben, jeweils
   mit Chlorameisensäureestern oder Chlorameisensäurethioestern der Formel (X)

   R²-M-CO-Cl (X)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(H) daß man Verbindungen der oben gezeigten Formel (I-1-c), in welcher A, B, R², W, M, V¹, V², V³, n und Z die oben angegebenen Bedeutungen haben und L für Schwefel steht, erhält, wenn man Verbindungen der oben gezeigten Formel (I-1-a), in welcher A, B, W, V¹, V², V³, n und Z die oben angegebenen Bedeutungen haben, jeweils
   α) mit Chlormonothioameisensäureestern oder Chlordithioameisensäureestern der Formel (XI) 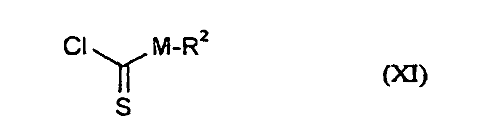 in welcher
      - M und R²: die oben angegebenen Bedeutungen haben,
      gegebenenfalls in Gegenwart eines Verdünnungsmittels und gegebenenfalls in Gegenwart eines Säurebindemittels umsetzt
      oder
   β) mit Schwefelkohlenstoff und anschließend mit Verbindungen der Formel (XII)

      R²-Hal (XII)

      in welcher
      - R²: die oben angegebene Bedeutung hat und
      - Hal: für Chlor, Brom oder Iod steht,
      gegebenenfalls in Gegenwart eines Verdünnungsmittels und gegebenenfalls in Gegenwart einer Base umsetzt,
(I) daß man Verbindungen der oben gezeigten Formel (I-1-d), in welcher A, B, R³, W, V¹, V², V³, n und Z die oben angegebenen Bedeutungen haben, erhält, wenn man Verbindungen der oben gezeigten Formel (I-1-a), in welcher A, B, W, V¹, V², V³, n und Z die oben angegebenen Bedeutungen haben, jeweils
   mit Sulfonsäurechloriden der Formel (XIII)

   R³-SO₂-Cl (XIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(J) daß man Verbindungen der oben gezeigten Formel (I-l-e), in welcher A, B, L, R⁴, R⁵, W, V¹, V², V³, n und Z die oben angegebenen Bedeutungen haben, erhält, wenn man Verbindungen der oben gezeigten Formel (I-1-a) in welcher A, B, W, V¹, V², V³, n und Z die oben angegebenen Bedeutungen haben, jeweils
   mit Phosphorverbindungen der Formel (XIV) 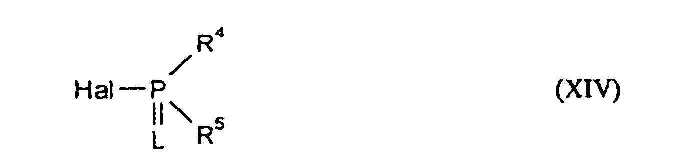 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(K) daß man Verbindungen der oben gezeigten Formel (I-1-f), in welcher A, B, E, W, V¹, V², V³, n und Z die oben angegebenen Bedeutungen haben, erhält, wenn man Verbindungen der Formel (I-1-a) in welcher A, B, W, V¹, V², V³, n und Z die oben angegebenen Bedeutungen haben, jeweils
   mit Metallverbindungen oder Aminen der Formeln (XV) oder (XVI)

   Me(OR¹⁰)ₜ (XV)

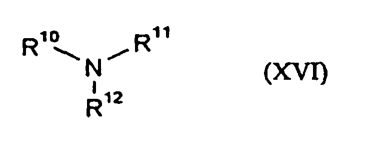

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(L) daß man Verbindungen der oben gezeigten Formel (I-1-g), in welcher A, B, L, R⁶, R⁷, W, V¹, V², V³, n und Z die oben angegebenen Bedeutungen haben, erhält, wenn man Verbindungen der oben gezeigten Formel (I-1-a), in welcher A, B, W, V¹, V², V³, n und Z die oben angegebenen Bedeutungen haben, jeweils
   α) mit Isocyanaten oder Isothiocyanaten der Formel (XVII)

      R⁶-N=C=L (XVII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XVIII) 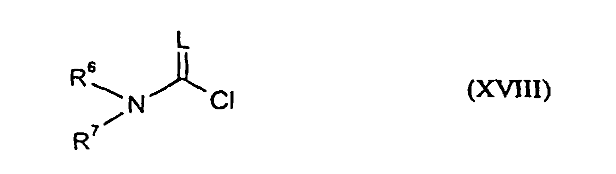 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen und darüber hinaus teilweise sehr gut pflanzenverträglich, insbesondere gegenüber Kulturpflanzen, sind.

Die erfindungsgemäßen Verbindungen sind durch die Formeln (I) bzw. (I-A), (I-B) und (I-C) allgemein definiert. Die Substituenten bzw. Bereiche der in der oben und nachstehend erwähnten Formeln aufgeführten Reste werden im folgenden erläutert:
- W: steht besonders bevorzugt für Wasserstoff, Nitro, Cyano, Fluor, Chlor, Brom, C₁-C₄-Alkyl, C₁-C₄-Alkoxy, C₁-C₄-Alkylthio, C₁-C₂-Halogenalkyl, C₁-C₂-Halogenalkoxy oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Benzyl oder Benzyloxy.
- X: steht besonders bevorzugt für Wasserstoff, Fluor, Chlor, Brom, C₁-C₄-Alkyl, C₁-C₄-Alkoxy, C₁-C₄-Alkylthio, C₁-C₂-Halogenalkyl, C₁-C₂-Halogenalkoxy, Cyano, Nitro oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Benzyl oder Benzyloxy.
- Y: steht besonders bevorzugt für Wasserstoff, Fluor, Chlor, Brom, C₁-C₄-Alkyl, C₁-C₄-Alkoxy, C₁-C₄-Alkylthio, C₁-C₂-Halogenalkyl, C₁-C₂-Halogenalkoxy, Cyano oder Nitro.
- Z: steht besonders bevorzugt für Fluor, Chlor, Brom, C₁-C₄-Alkyl, C₁-C₄-Alkoxy, C₁-C₄-Alkylthio, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄-Alkoxy, C₁-C₂-Halogenalkyl, C₁-C-Halogenalkoxy, Nitro oder Cyano substituiertes Phenoxy oder Benzyloxy.
- n: steht besonders bevorzugt für 0 oder 1.
- Het: steht besonders bevorzugt für eine Gruppe 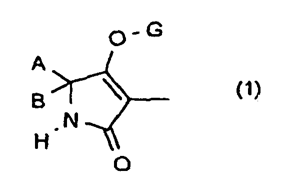
- A: steht besonders bevorzugt für Wasserstoff, für jeweils gegebenenfalls durch Fluor oder Chlor substituiertes C₁-C₁₀-Alkyl, C₂-C₆-Alkenyl, C₁-C₈-Alkoxy-C₁-C₆-alkyl, Poly-C₁-C₆-alkoxy-C₁-C₆-alkyl oder C₁-C₈-Alkylthio-C₁-C₆-alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl.
- B: steht besonders bevorzugt für Wasserstoff, C₁-C₁₀-Alkyl oder C₁-C₆-Alkoxy-C₁-C₄-alkyl oder
- A, B: und das Kohlenstoffatom an das sie gebunden sind, stehen besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch C₁-C₆-Alkyl oder C₁-C₆-Alkoxy substituiert sind oder
- A, B: und das Kohlenstoffatom, an das sie gebunden sind, stehen besonders bevorzugt für C₅-C₆-Cycloalkyl, welches durch eine gegebenenfalls ein oder zwei Sauerstoff- oder Schwefelatome enthaltende Alkylendiyl- oder durch eine Alkylendioxy- oder durch eine Alkylendithioyl-Gruppe substituiert ist, die mit dem Kohlenstoffatom, an das sie gebunden ist, einen weiteren fünf- bis siebengliedrigen Ring bildet oder
- A, B: und das Kohlenstoffatom, an das sie gebunden sind, stehen besonders bevorzugt für C₃-C₆-Cycloalkyl oder C₅-C₆-Cycloalkenyl, in dem zwei Kohlenstoffatome durch jeweils gegebenenfalls durch C₁-C₄-Alkyl oder C₁-C₄-Alkoxy substituiertes C₃-C₅-Alkandiyl, C₃-C₅-Alkendiyl oder Butadiendiyl miteinander verbunden sind, worin jeweils gegebenenfalls eine Methylengruppe durch Sauerstoff oder Schwefel ersetzt ist.
- G: steht besonders bevorzugt für Wasserstoff (a) oder für eine der Gruppen 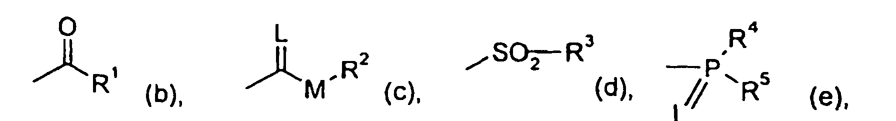 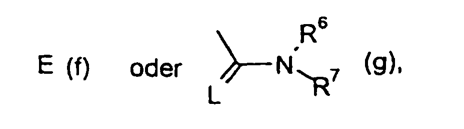 in welchen
E für ein Metallionäquivalent oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls durch Fluor oder Chlor substituiertes C₁-C₆-Alkyl oder jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl.
- R⁴ und R⁵: stehen unabhängig voneinander besonders bevorzugt für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.
- W: steht ganz besonders bevorzugt für Wasserstoff, Fluor, Chlor, Brom, Methyl, Ethyl, Methoxy, Ethoxy oder Trifluormethyl.
- X: steht ganz besonders bevorzugt für Wasserstoff, Fluor, Chlor, Brom, Methyl, Ethyl, Methoxy, Ethoxy oder Trifluormethyl.
- Y: steht ganz besonders bevorzugt für Wasserstoff, Fluor, Chlor, Brom, Methyl, Ethyl, Methoxy, Ethoxy oder Trifluormethyl.
- Z: steht ganz besonders bevorzugt für Fluor, Chlor, Brom, Methyl, Ethyl, Methoxy, Ethoxy oder Trifluormethyl.
- n: steht ganz besonders bevorzugt für 0 oder 1.
- Het: steht ganz besonders bevorzugt für eine Gruppe 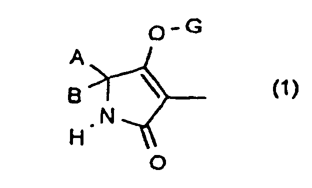
- A: steht ganz besonders bevorzugt für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄-alkyl, Poly-C₁-C₄-alkoxy-C₁-C₄-alkyl oder C₁-C₆-Alkylthio-C₁-C₄-alkyl, oder für gegebenenfalls durch Fluor, Chlor, Methyl oder Methoxy substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl oder Trifluormethoxy substituiertes Phenyl, Pyridyl oder Benzyl.
- B: steht ganz besonders bevorzugt für Wasserstoff, C₁-C₈-Alkyl oder C₁-C₄-Alkoxy-C₁-C₂-alkyl oder
- A, B: und das Kohlenstoffatom an das sie gebunden sind, stehen ganz besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch Methyl, Ethyl, n-Propyl, iso-Propyl, Butyl, iso-Butyl, sec.-Butyl, tert.-Butyl, Methoxy, Ethoxy, n-Propoxy, iso-Propoxy, Butoxy, iso-Butoxy, sek.-Butoxy oder tert.-Butoxy substituiert sind oder
- A, B: und das Kohlenstoffatom, an das sie gebunden sind, stehen ganz besonders bevorzugt für C₅-C₆-Cycloalkyl, welches durch eine gegebenenfalls ein Sauerstoff- oder Schwefelatom enthaltende Alkylendiyl- oder durch eine Alkylendioxy-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, stehen ganz besonders bevorzugt für C₃-C₆-Cyctoalkyl oder C₅-C₆-Cycloalkenyl, in dem zwei Kohlenstoffatome durch C₃-C₄-Alkandiyl, C₃-C₄-Alkendiyl oder Butadiendiyl miteinander verbunden sind, worin jeweils gegebenenfalls eine Methylengruppe durch Sauerstoff oder Schwefel ersetzt ist.
- G: steht ganz besonders bevorzugt für Wasserstoff (a) oder für eine der Gruppen 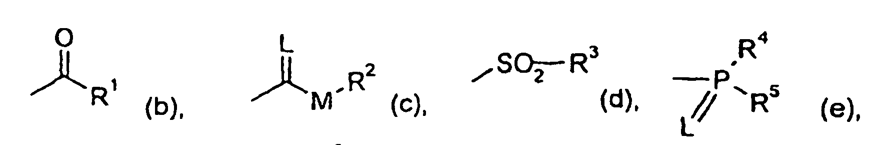 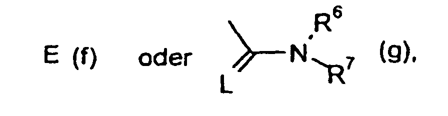 in welchen
E für ein Metallionäquivalent oder ein Ammoniumion steht,
L für Sauerstoff oder Schwefel steht und
M für Sauerstoff oder Schwefel steht.
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarate Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R³: steht ganz besonders bevorzugt für gegebenenfalls durch Fluor oder Chlor substituiertes Methyl, Ethyl, Propyl, iso-Propyl, n-Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl.
- R⁴ und R⁵: stehen unabhängig voneinander ganz besonders bevorzugt für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.

Dabei gilt immer, daß W und Z nur dann gemeinsam mit den Kohlenstoffatomen, an die sie gebunden sind, einen Ring bilden können, wenn diese unmittelbar benachbart sind.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Eine besonders bevorzugte Gruppe von Verbindungen der Formel (I) sind solche, in welchen V¹ für Stickstoff steht.

Eine weitere, besonders bevorzugte Gruppe von Verbindungen der Formel (I) sind solche, in welchen V² für Stickstoff steht.

Eine weitere, besonders bevorzugte Gruppe von Verbindungen der Formel (I) sind solche, in welchen V³ für Stickstoff steht.

Besonders bevorzugt sind weiterhin Verbindungen der Formel (I), in welchen G für Wasserstoff (a) oder eine der Gruppen 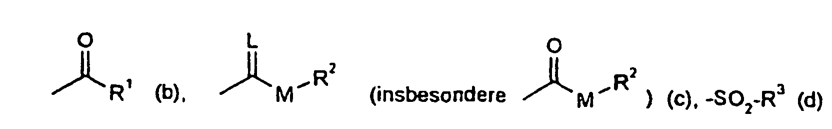 oder 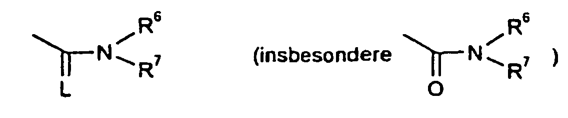 steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzeinen seien außer den bei den Herstellungsbeispielen genannten Verbindungen die folgenden Verbindungen der Formel (I-1-a) genannt:

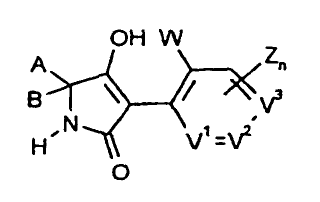

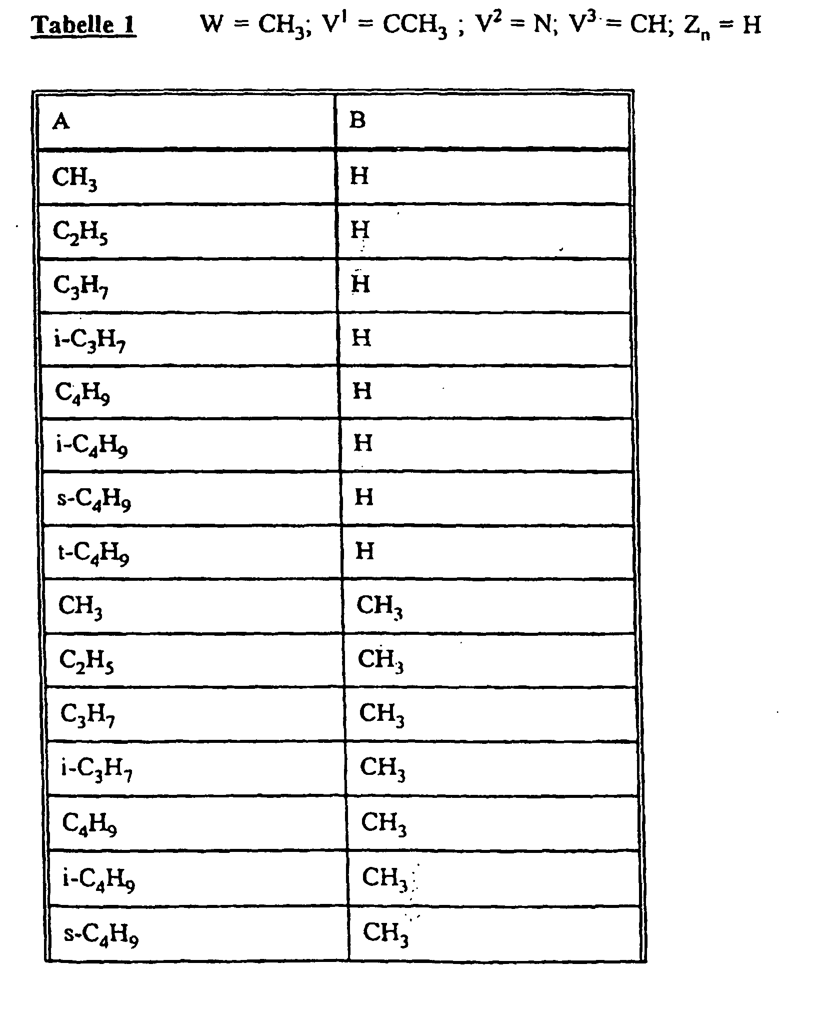

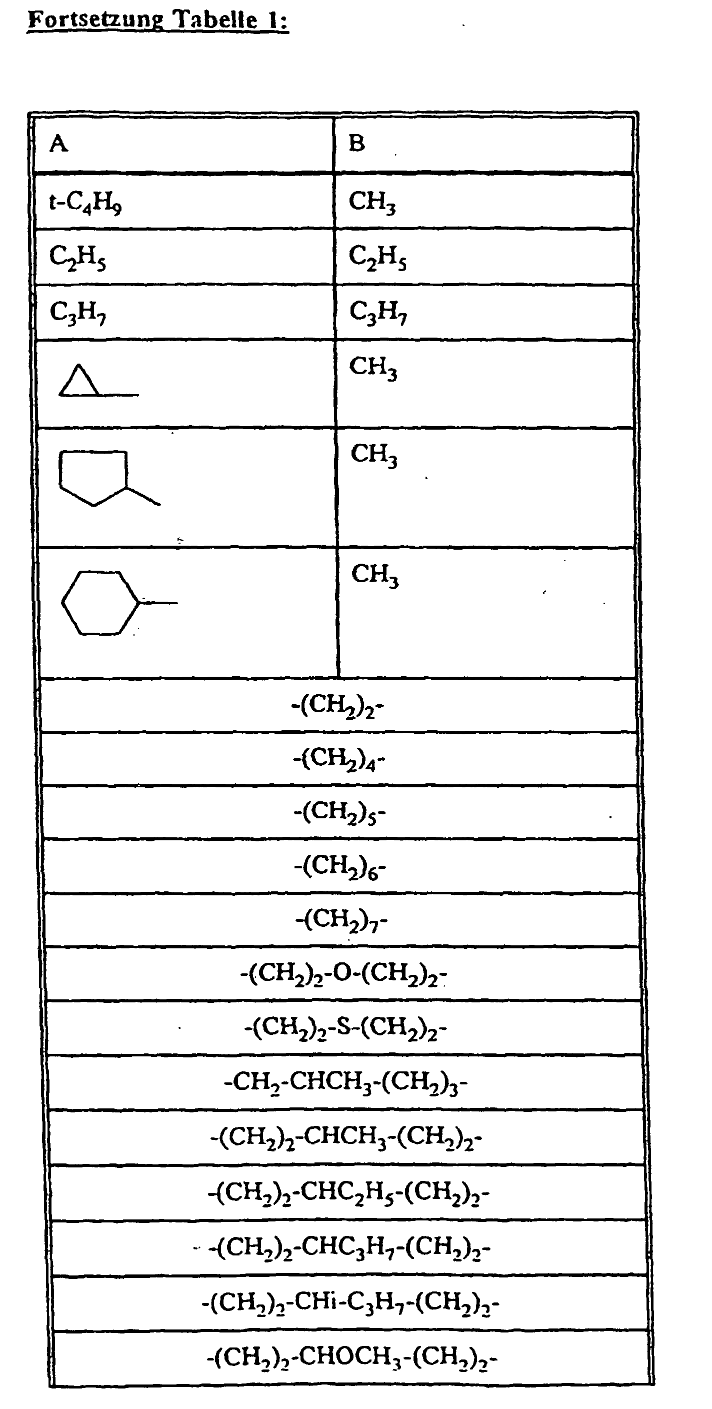

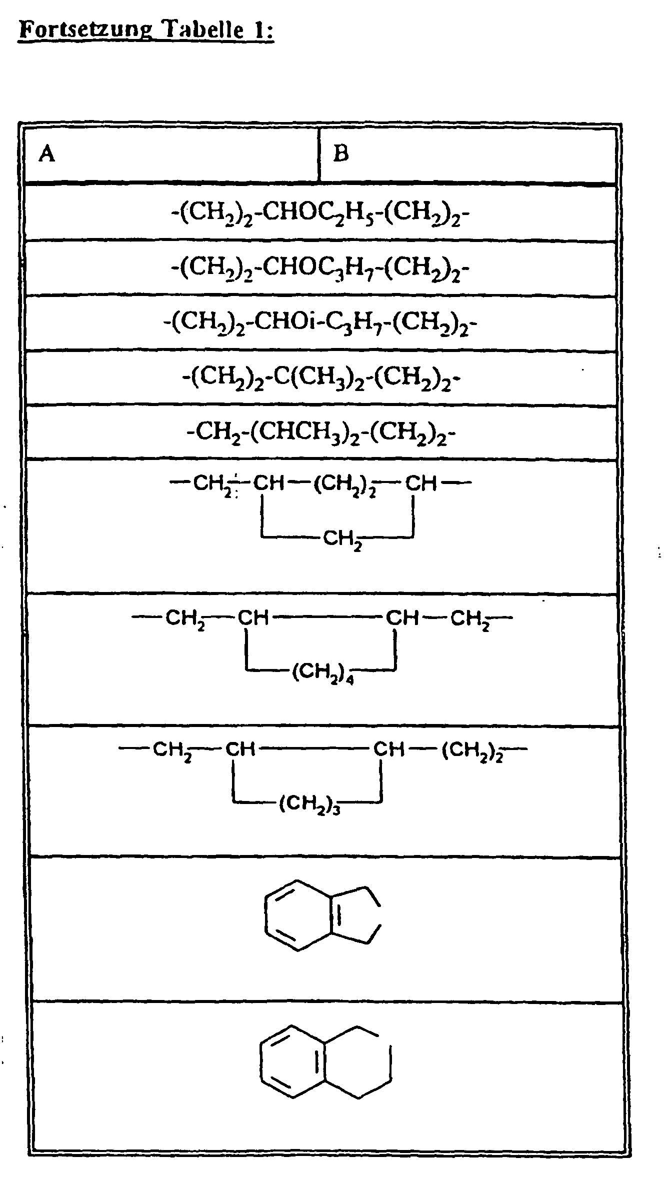

- **Tabelle 2:**: A und B haben die gleiche Bedeutung wie in Tabelle 1 mit W = CH₃; V¹ = CCI; V² = N; V³ = CH; Zₙ = H
- **Tabelle 3:**: A und B haben die gleiche Bedeutung wie in Tabelle 1 mit W = CH₃; V¹ = CCH₃ V² = N; V³ = CCH₃; Zₙ = H
- **Tabelle 4:**: A und B haben die gleiche Bedeutung wie in Tabelle 1 mit W=H; V¹ =CCI; V² = N; V³ = CH; Zₙ = H
- **Tabelle 5:**: A und B haben die gleiche Bedeutung wie in Tabelle 1 mit W = CH₃; V¹ = N; V² = CH; V³ = CCH₃; Zₙ = H
- **Tabelle 6:**: A und B haben die gleiche Bedeutung wie in Tabelle 1 mit W = Cl; V¹ = N; V² = CH; V³ = CCI; Zₙ = H
- **Tabelle 7:**: A und B haben die gleiche Bedeutung wie in Tabelle 1 mit W = Cl; V¹ = N; V² = CH; V³ = CCH₃; Zₙ = H
- **Tabelle 8:**: A und B haben die gleiche Bedeutung wie in Tabelle 1 mit W = CH₃; V¹ = N; V² = CH; V³ = CCI, Zₙ = H
- **Tabelle 9:**: A und B haben die gleiche Bedeutung wie in Tabelle 1 mit W = CH₃; V¹ = CH; V² = CH; V³ = N, Zₙ = H
- **Tabelle 10:**: A und B haben die gleiche Bedeutung wie in Tabelle 1 mit W = H; V¹ = C-Cl; V² = N; V³ = C-Cl; Zₙ = H
- **Tabelle 11:**: A und B haben die gleiche Bedeutung wie in Tabelle 1 mit W = Cl; V¹ = CH; V² = N; V³ = C-Cl; Zₙ = H

Verwendet man gemäß Verfahren (A) N-[3-(6-Chlor-2,4-dimethyl)-pyridylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

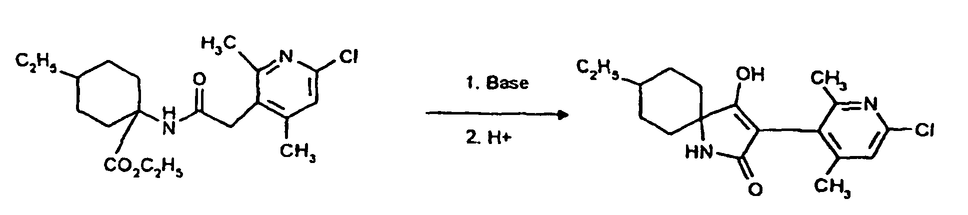

Verwendet man gemäß Verfahren (Fα) 3-[4-(3-Chlor-5-methyl)-pyridyl]-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

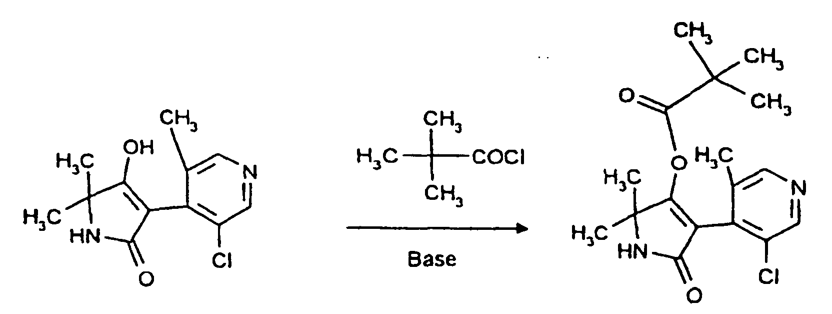

Verwendet man gemäß Verfahren (F) (Variante β) 3-[2-(3-Chlor)-pyridil]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

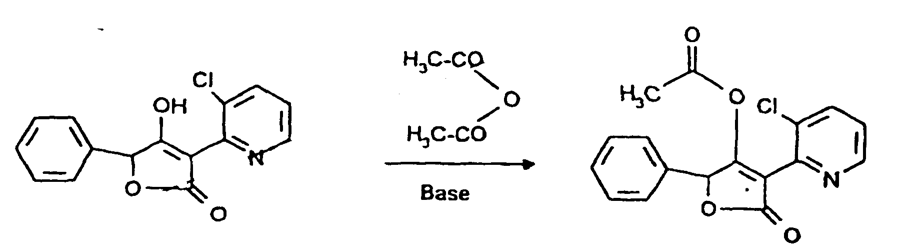

Verwendet man gemäß Verfahren (G) 8-[2-(3,5-Dichlor-4-methyl)-pyridyl]-5,5-pentamethylen-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

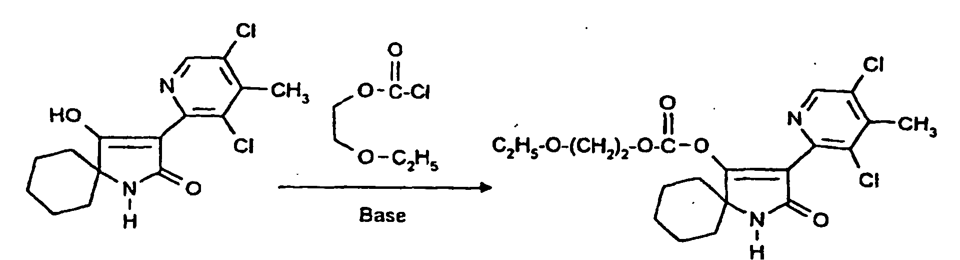

Verwendet man gemäß Verfahren (H), (Variante α) 3-[2-(3-Brom-5,6-dimethyl)-pyridil]-4-hydroxy-6-(3-pyridil)-pyron und Chlormonothioameisensäuremethylester als Ausgangsprodukte, so kann der Reaktionsverlauf folgendermaßen wiedergegeben werden:

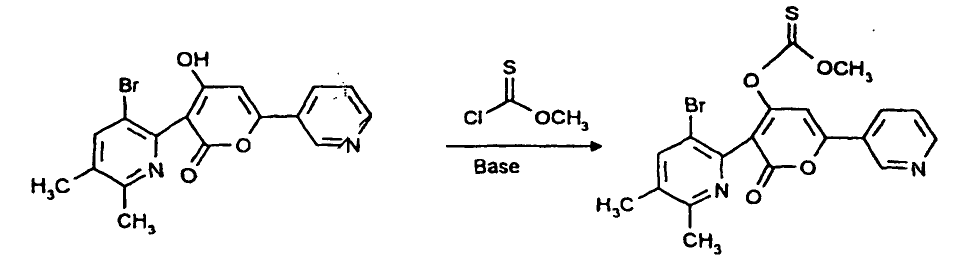

Verwendet man gemäß Verfahren (H), (Variante β) 5-[4-(2-Chlor-3,5-dimethyl)-pyridil]-6-hydroxy-2-(4-chlorphenyl)-thiazin-4-on, Schwefelkohlenstoff und Methyliodid als Ausgangskomponenten, so kann der Reaktionsverlauf wie folgt wiedergegeben werden:

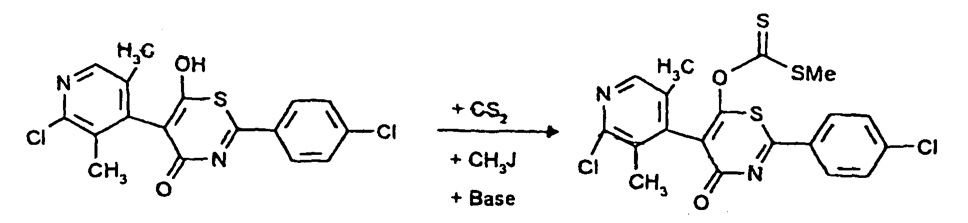

Verwendet man gemäß Verfahren (I) 2-[3-(2,4,6-Trimethyl)-pyridyl]-5,5[-(3-methyl)pentamethylen]-pyrrolidin-2,4-dion und Methansulfonsäurechlorid als Ausgangsprodukte, so kann der Reaktionsverlauf durch folgendes Reaktionsschema wiedergegeben werden:

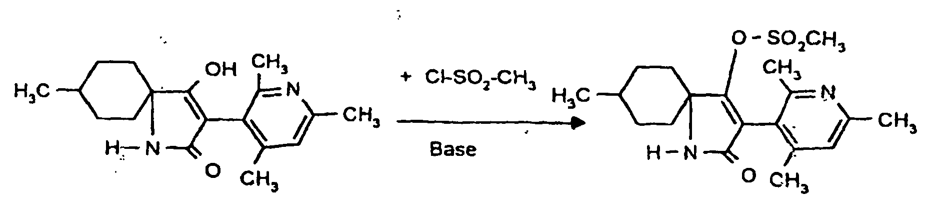

Verwendet man gemäß Verfahren (J) 2-[4-(3-Chlor-5-methyl)-pyridyl]-4-hydroxy-5-methyl-6-(2-pyridil)-pyron und Methanthio-phosphonsäurechlorid-(2,2,2-trifluorethylester) als Ausgangsprodukte, so kann der Reaktionsverlauf durch folgendes Reaktionsschema wiedergegeben werden:

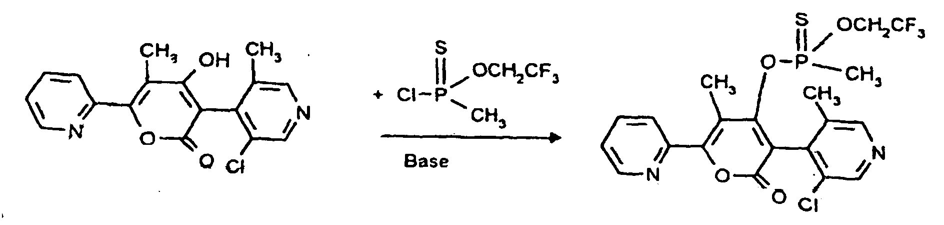

Verwendet man gemäß Verfahren (K) 3-[3-(2,6-Dichlor)-4-methylpyridyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

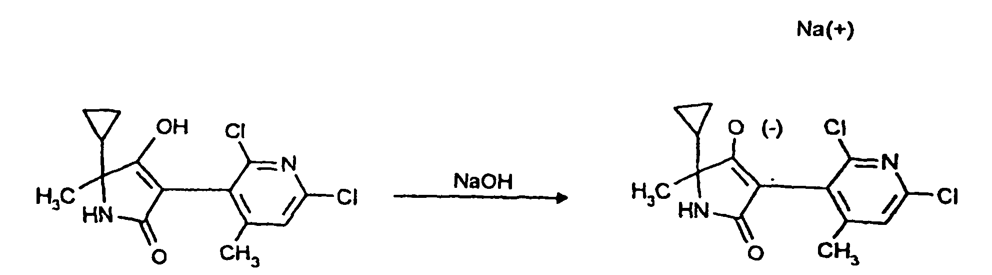

Verwendet man gemäß Verfahren (L) (Variante α) 3-[3-(2-Chlor-6-brom-5-methyl)-pyridyl]-4-hydroxy-5,5-tetramethylen-Δ³-dihydro-furan-2-on und Ethylisocya-nat als Ausgangsprodukte, so kann der Reaktionsverlauf durch folgendes Reaktionsschema wiedergegeben werden:

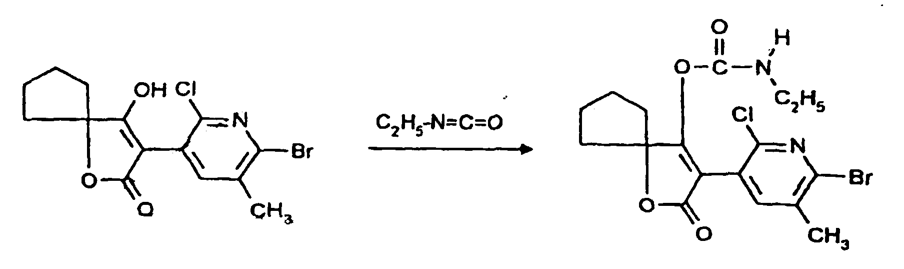

Verwendet man gemäß Verfahren (L) (Variante β) 3-[4-(3-Chlor-5-methyl)-pyridyl]-5-methyl-pyrrolidin-2,4-dion und Dimethylcarbamidsäurechlorid als Ausgangsprodukte, so kann der Reaktionsverlauf durch folgendes Schema wiedergegeben werden:

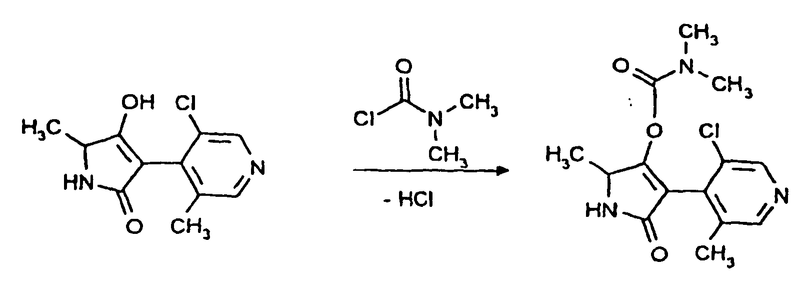

Die beim erfindungsgemäßen Verfahren (A) als Ausgangsstoffe benötigten Verbindungen der Formel (II) 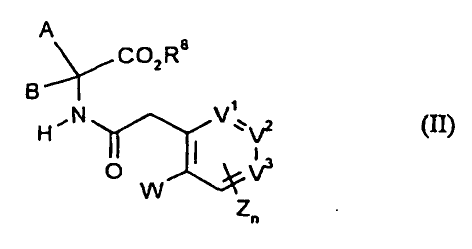 in welcher
A, B, W, X, Z, V¹, V², V³, n und R⁸ die oben angegebenen Bedeutungen haben,
sind teilweise neu.

Man erhält die Acylaminosäureester der Formel (II) beispielsweise, wenn man Aminosäurederivate der Formel (XIX) 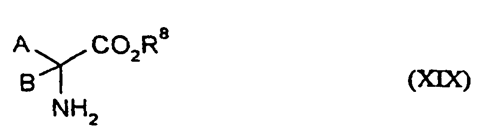 in welcher
- A, B und R⁸: die oben angegebenen Bedeutungen haben,
mit substituierten Pyridylessigsäurehalogeniden der Formel (XX) 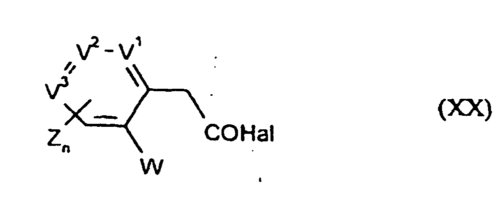 in welcher
- W, V¹, V², V³, n und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXI) 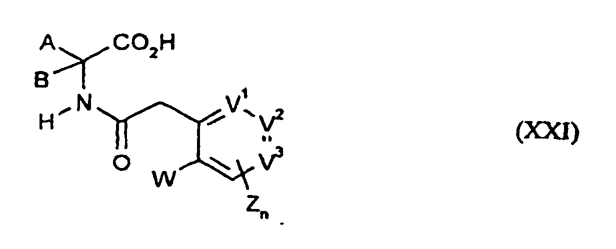 in welcher
- A, B, W, V¹, V², V³, n und Z: die oben angegebenen Bedeutungen haben,
verestert (Chem. Ind. (London) 1568 (1968)).

Die Verbindungen der Formel (XXI) 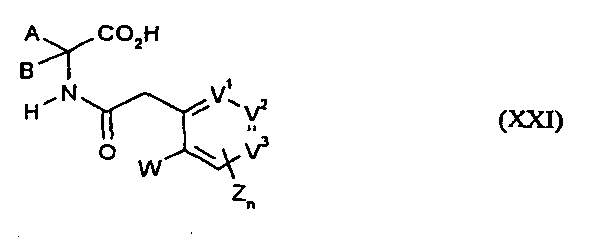 in welcher
- A, B, W, V¹, V², V³, n und Z: die oben angegebenen Bedeutungen haben,
erhält man, wenn man Aminosäuren der Formel (XXII) 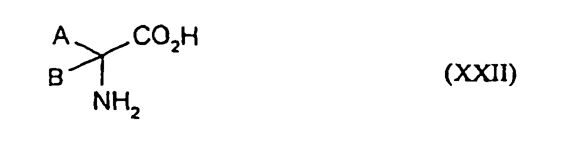 in welcher
- A und B: die oben angegebenen Bedeutungen haben,
mit substituierten Pyridylessigsäurehalogeniden der Formel (XX) 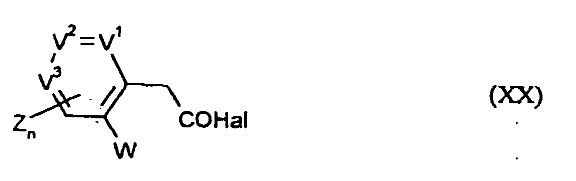 in welcher
- W, V¹, V², V³,: n und Z die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XX) sind teilweise neu. Sie lassen sich nach bekannten Verfahren herstellen.

Man erhält die Verbindungen der Formel (XX) beispielsweise, indem man substituierte Pyridylessigsäuren der Formel (XXIII) 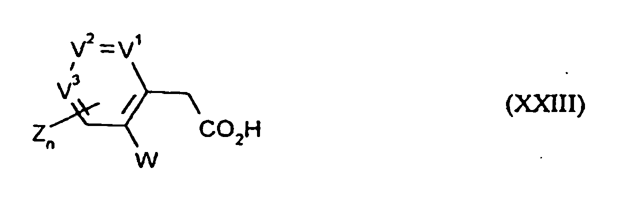 in welcher
- W, V¹, V², V³, n und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXIII) sind teilweise neu. Sie lassen sich nach literaturbekannten Verfahren herstellen (siehe z.B. Organikum 15. Auflage, S. 533, VEB Deutscher Verlag der Wissenschaften, Berlin 1977 und die Herstellungsbeispiele). Man erhält die Verbindungen der Formel (XXIII) beispielsweise, indem man substituierte Pyridylessigsäureester der Formel (XXIV) 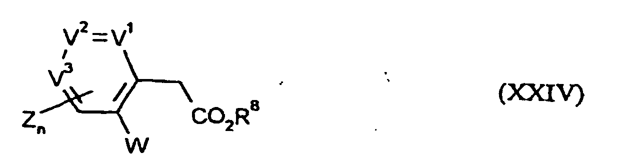 in welcher
- W, V¹, V², V³, n, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXIV) sind teilweise neu. Sie lassen sich nach im Prinzip bekannten Verfahren herstellen.

Man erhält die Verbindungen der Formel (XXIV) beispielsweise, indem man substituierte 1,1,1- Trichlor-2-pyridylethane der Formel (XXV) 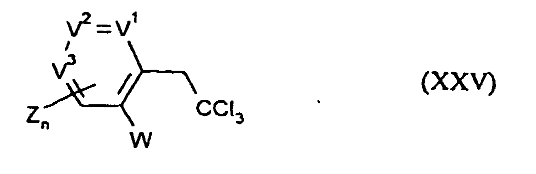 in welcher
- W, V¹, V², V³, n und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE 3 314 249).

Die Verbindungen der Formel (XXV) lassen sich nach im Prinzip bekannten Verfahren herstellen.

Man erhält die Verbindungen der Formel (XXV) beispielsweise, wenn man Aminopyridine der Formel (XXVI) 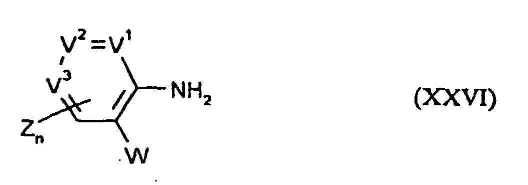 in welcher
- W, V¹, V², V³, n und Z: die oben angegebene Bedeutung haben,
in Gegenwart eines Alkylnitrits der Formel (XXVII)

R²¹-ONO (XXVII)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formeln (XXVI) und (XXVII) sind bekannte Verbindungen der Organischen Chemie. Kupfer(II)-chlorid und Vinylidenchlorid sind lange bekannt und käuflich.

Die Verbindungen der Formeln (XIX) und (XXII) sind teilweise bekannt und/oder lassen sich nach bekannten Verfahren darstellen (siehe z.B. Compagnon, Ann. Chim. (Paris) [14] 5, S. 11-22, 23-27 (1970)).

Die substituierten cyclischen Aminocarbonsäuren der Formel (XXIIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 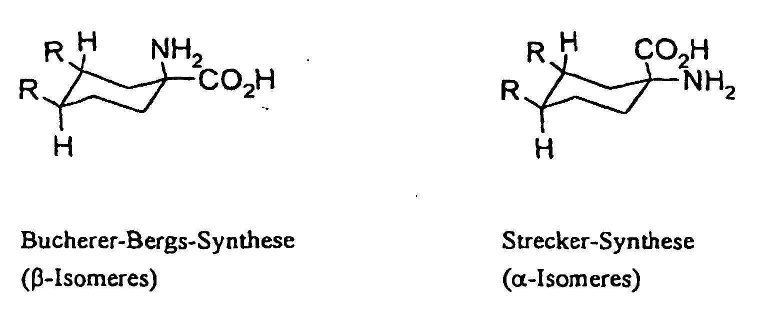 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 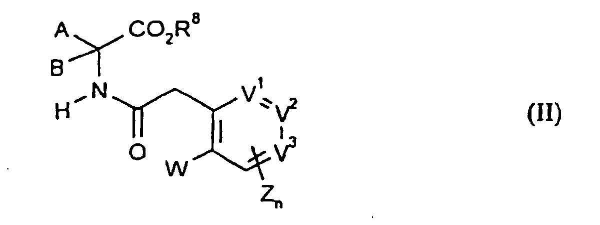 in welcher
- A, B, W, V¹, V², V³, n, Z und R⁸: die oben angegebenen Bedeutungen haben,
herstellen, wenn man Aminonitrile der Formel (XXVIII) 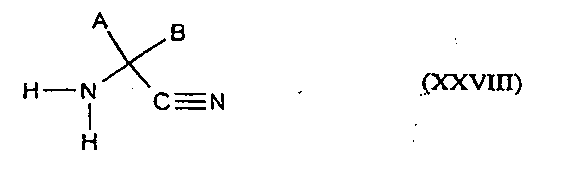 in welcher
- A und B: die oben angegebenen Bedeutungen haben,
mit substituierten Pyridylessigsäurehalogeniden der Formel (XX) 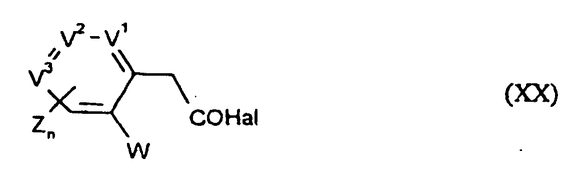 in welcher
- W, V¹, V², V³, n, Z und Hal: die oben angegebenen Bedeutungen haben,
zu Verbindungen der Formel (XXIX) 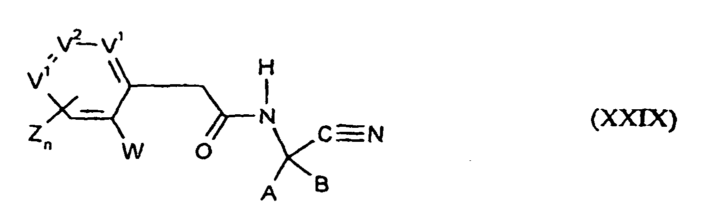 in welcher
- A, B, W, V¹, V², V³, n und Z: die oben angegebenen Bedeutungen haben,
umsetzt,
und diese änschließend einer sauren Alkoholyse unterwirft.

Die zur Durchführung der erfindungsgemäßen Verfahren (F), (G), (H), (I), (J), (K) und (L) außerdem als Ausgangsstoffe benötigten Säurehalogenide der Formel (VIII), Carbonsäureanhydride der Formel (IX), Chlorameisensäureester oder Chlorameisensäurethioester der Formel (X), Chlormonothioameisensäureester oder Chlordithioameisensäureester der Formel (XI), Alkylhalogenide der Formel (XII), Sulfonsäurechloride der Formel (XIII), Phosphorverbindungen der Formel (XIV) und Metallhydroxide, Metallalkoxide oder Amine der Formel (XV) und (XVI) und Isocyanate der Formel (XVII) und Carbamidsäurechloride der Formel (XVIII) sind allgemein bekannte Verbindungen der organischen bzw. anorganischen Chemie.

Die Verbindungen der Formeln (V), (Va), (VII) bis (XIX), (XXII), (XXVIII), (XXX), (XXXI), (XXXII), sind darüber hinaus aus den eingangs zitierten Patentanmeldungen bekannt und/oder lassen sich nach den dort angegebenen Methoden herstellen.

Das Verfahren (A) ist dadurch,gekennzeichnet, daß man Verbindungen der Formel (II), in welcher A, B, W, V¹, V², V³, n, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -70°C und 150°C, vorzugsweise zwischen -50°C und 100°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (Fα) ist dadurch gekennzeichnet, daß man Verbindungen der Formel (I-1-a) mit Carbonsäurehalogeniden der Formel (VIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Fα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Fα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fα) werden die Ausgangsstoffe der Formel (I-1-a) und das Carbonsäurehalogenid der Formel (VIII) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Fβ) ist dadurch gekennzeichnet, daß man Verbindungen der Formel (I-1-a) mit Carbonsäureanhydriden der Formel (IX) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Fβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Fβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fβ) werden die Ausgangsstoffe der Formel (I-1-a) und das Carbonsäureanhydrid der Formel (IX) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (G) ist dadurch gekennzeichnet, daß man Verbindungen der Formel (I-1-a) mit Chlorameisensäureestern oder Chlorameisensäurethiolestern der Formel (X) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (G) alle üblichen Säureakzeptoren in Betracht. Vorzugsweise verwendbar sind tertiäre Amine, wie Triethylamin. Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (G) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (G) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (G) wird im allgemeinen unter Normaldruck durchgeführt.

Bei der Durchführung des erfindungsgemäßen Verfahrens (G) werden die Ausgangsstoffe der Formel (I-1-a) und der entsprechende Chlorameisensäureester bzw. Chlorameisensäurethiolester der Formel (X) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (H) ist dadurch gekennzeichnet, daß man Verbindungen der Formel (I-1-a) mit (Hα) Verbindungen der Formel (XI) in Gegenwart eines Verdünnungsmittels und gegebenenfalls in Gegenwart eines Säurebindemittels oder (Hβ) Schwefelkohlenstoff und anschließend mit Alkylhalogeniden der Formel (XII) gegebenenfalls in Gegenwart eines Verdünnungsmittels und gegebenenfalls in Gegenwart einer Base umsetzt.

Beim Herstellungsverfahren (Hα) setzt man pro Mol Ausgangsverbindung der Formeln (I-1-a) bis (I-5-a) ca 1 Mol Chlormonothioameisensäureester bzw. Chlordithioameisensäureester der Formel (X1) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder .. Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Hβ) setzt man pro Mol Ausgangsverbindungen der Formel (I-1-a) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus den Verbindungen der Formel (I-1-a) durch Zusatz einer Base (wie z.B. Kaliumtertiärbutylat oder Natriumhydrid) das entsprechende Salz herzustellen. Man setzt die Verbindungen (I-1-a) jeweils so lange mit Schwefelkohlenstoff um, bis die Bildung der Zwischenverbindung abgeschlossen ist, z.B. nach mehrstündigem Rühren bei Raumtemperatur.

Als Basen können beim Verfahren (Hβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise sind verwendbar aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Die weitere Umsetzung mit dem Alkylhalogenid der Formel (XII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Das erfindungsgemäße Verfahren (I) ist dadurch gekennzeichnet, daß man Verbindungen der Formel (1-1-a) mit Sulfonsäurechloriden der Formel (XIII) gegebenenfalls in Gegenwart eines Verdünnungsmittels und gegebenenfalls in Gegenwart eines Säurebindemittels umsetzt.

Beim Herstellungsverfahren (1) setzt man pro Mol Ausgangsverbindung der Formel (I-1-a) ca. 1 Mol Sulfonsäurechlorid der Formel (XIII) bei -20 bis 150°C, vorzugsweise bei 0 bis 70°C um.

Das Verfahren (1) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (J) ist dadurch gekennzeichnet, daß man Verbindungen der Formel (I-1-a) jeweils mit Phosphorverbindungen der Formel (XIV) gegebenenfalls in Gegenwart eines Verdünnungsmittels und gegebenenfalls in Gegenwart eines Säurebindemittels umsetzt.

Beim Herstellungsverfahren (J) setzt man zum Erhalt von Verbindungen der Formel (I-1-e) auf 1 Mol der Verbindungen (I-1-a), 1 bis 2, vorzugsweise 1 bis 1,3 Mol der Phosphorverbindung der Formel (XIV) bei Temperaturen zwischen -40°C und 150°C. vorzugsweise zwischen -10 und 110°C um.

Das Verfahren (J) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (K) ist dadurch gekennzeichnet, daß man Verbindungen der Formel (I-1-a) mit Metallhydroxiden bzw. Metallalkoxiden der Formel (XV) oder Aminen der Formel (XVI), gegebenenfalls in Gegenwart eines Verdünnungsmittels, umsetzt.

Als Verdünnungsmittel können bei dem erfindungsgemäßen Verfahren (K) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (K) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (L) ist dadurch gekennzeichnet, daß man Verbindungen der Formel (I-1-a) mit (Lα) Verbindungen der Formel (XVII) gegebenenfalls in Gegenwart eines Verdünnungsmittels und gegebenenfalls in Gegenwart eines Katalysators oder (Lβ) mit Verbindungen der Formel (XVIII) gegebenenfalls in Gegenwart eines Verdünnungsmittels und gegebenenfalls in Gegenwart eines Säurebindemittels umsetzt.

Bei Herstellungsverfahren (Lα) setzt man pro Mol Ausgangsverbindung der Formel (I-1-a) ca. 1 Mol Isocyanat der Formel (XVII) bei 0 bis 100°C, vorzugsweise bei 20 bis 50°C um.

Das Verfahren (Lα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Lβ) setzt man pro Mol Ausgangsverbindung der Formel (I-1-a) ca. 1 Mol Carbamidsäurechlorid der Formel (XVIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica. Acheta domesticus, Gryllotalpa spp., Locusta migratoria migratorioides. Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp . Euscelis bilobatus, Nephotettix cincticeps, Lecanium corni, Saissetia oleae, Laodelphax striatellus, Nilaparvata lugens, Aonidiella aurantii, Aspidiotus hederae. Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderrna spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) oder gegen die Raupen der Kohlschabe (Plutella maculipennis) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver. Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz. Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt. Molybdan und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden. Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Funnecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasutfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexatlumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos. Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silatluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB. 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z B. Chloridazon und Norflurazon, Carbamate, wie z.B Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.% Wirkstoff, vorzugsweise von 0,0001 bis 1 Gew.% betragen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinarmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlehutomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp.. Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp, Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp, Sternostoma spp., Varroa spp

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.; Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injekuonen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann, man die Wirkstoffe der Formel (l) als Formulierungen (beispielsweise Pulver, Emulsionen, fließFähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstoren.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwanze wie

Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw, dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C. vorzugsweise oberhalb 45°C, eingesetzt. Als derartige schwerflüchtige, wasserunlosliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz. Siliconharz, trocknende pflanzliche und/oder trocknende Öle und/oder physikalisch trocknende Bindemittel auf der Basis eines Natur- und/oder Kunstharzes verwendet

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden.. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusatzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Herstellungsbeispiele

### Beispiel (I-B-1-a-1)

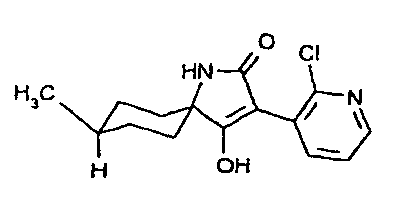

Zu 8,82 g (0,075 mol) Kalium-tert.-butylat in 30 ml DMF tropft man bei 0 bis 10°C 11,3 g der Verbindung gemäß Beispiel (II-B-1) in 22 ml DMF und rührt bei Raumtemperatur. Der Reaktionsfortschritt wird mittels Dünnschichtchromatographie (DC) verfolgt. Nach beendeter Reaktion gibt man 250 ml Eiswasser zu und säuert bei 0 bis 10°C mit konzentrierter Salzsäure auf pH 2 an, saugt ab, wäscht mit Wasser, trocknet und kocht zur Reinigung mit Methyl-tert.-butylether (MTB-Ether)/n-Hexan auf. Ausbeute 7,90 g (77 % der Theorie) Fp. >220°C.

Analog zu Beispiel I-B-1-a-1 bzw. gemäß den allgemeinen Angaben zur Herstellung der Verbindungen der Formel (I-1-a) wurden folgende Verbindungen der Formel (I-1-a) erhalten:

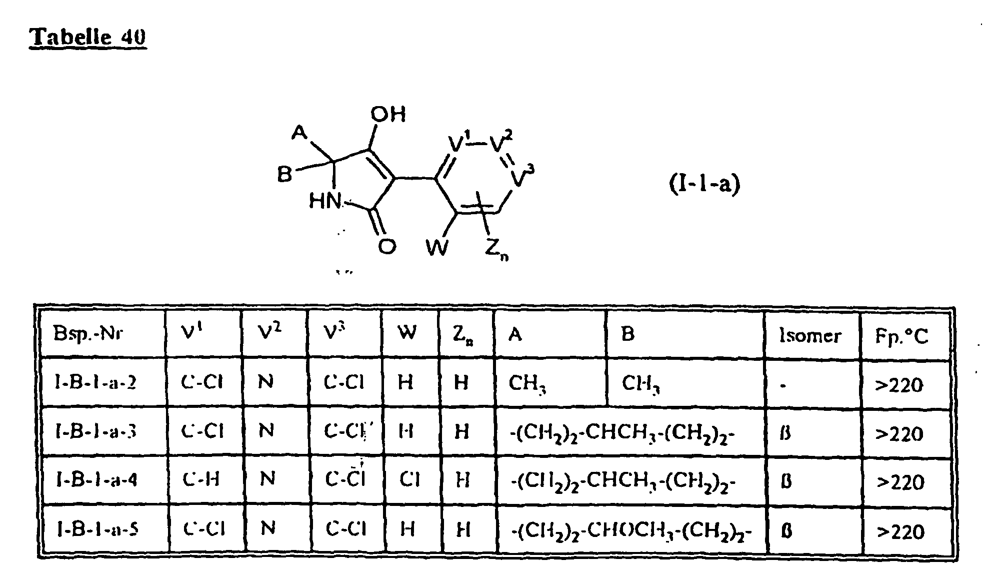

### Beispiel (I-B-1-b-1)

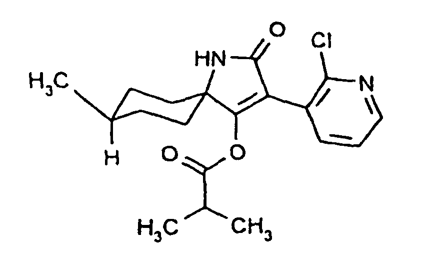

Zu 2,34 g der Verbindung gemäß Beispiel (I-B-1-a-1) und 1,7 ml (12 mmol) Triethylamin in 50 ml wasserfreiem Essigsäureethylester tropft man bei Rückflußtemperatur 1,3 ml (0,012 mol) Isobuttersäurechlorid in 5 ml wasserfreiem Essigsäureethylester, rührt unter Rückfluß und verfolgt den Reaktionsfortschritt mittels DC. Nach beendeter Reaktion wird eingeengt, in Methylenchlorid aufgenommen, 2 mal mit 50 ml 0,5 N NaOH gewaschen, getrocknet und eingeengt. Der Rückstand wird aus MTB-Ether/n-Hexan umkristallisiert.
Ausbeute 1,5 g (51 % der Theorie), Fp. 204°C.

Analog zu Beispiel I-B-1-b-1 bzw. gemäß den allgemeinen Angaben zur Herstellung wurden folgende Verbindungen der Formel (I-1-b) erhalten:

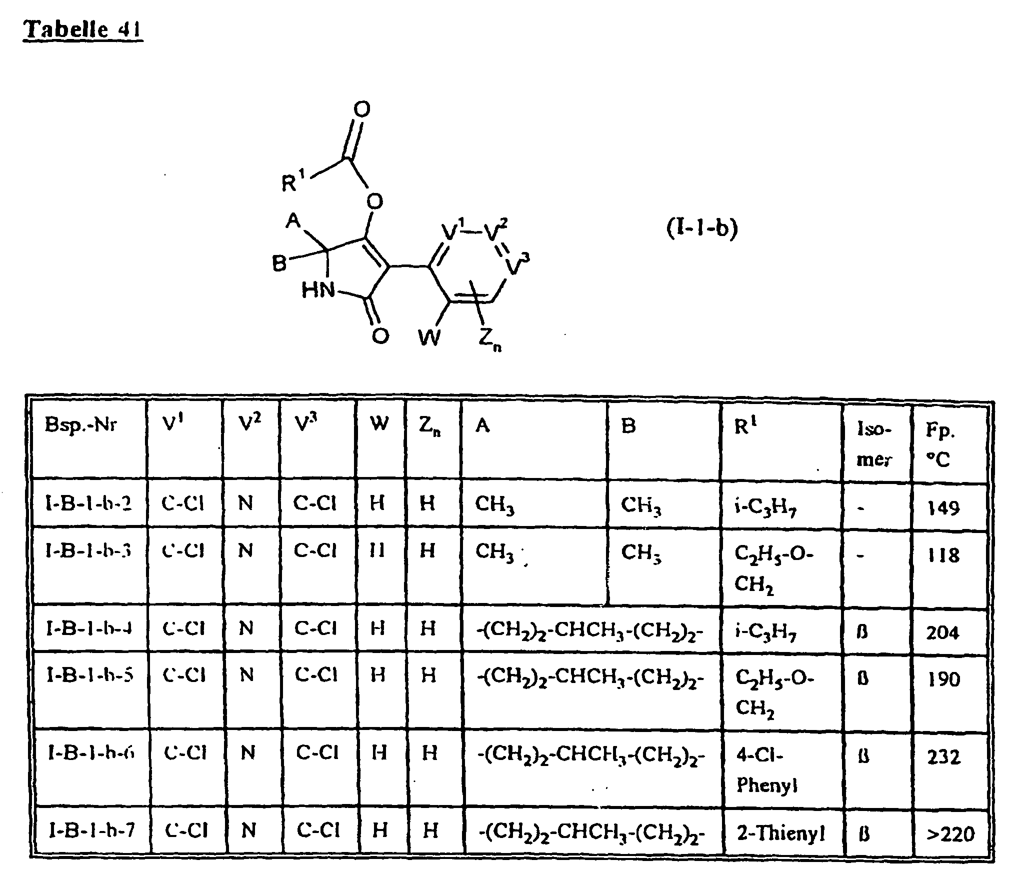

### Beispiel (I-B-1-c-1)

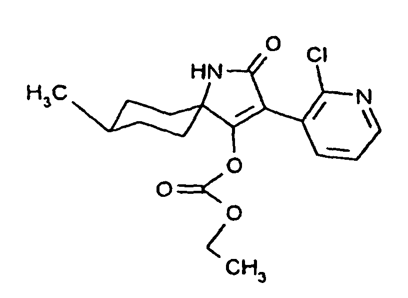

Zu 2,34 g der Verbindung gemäß Beispiel (I-B-1-a-1) und 1,2 ml (0,008 mol) Triethylamin in 50 ml wasserfreiem Methylenchlorid tropft man bei 0 bis 10°C 0,8 ml (0.008 mol) Chlorameisensäureethylester in 5 ml wasserfreiem Methylenchlorid. Man rührt bei Raumtemperatur und verfolgt den Reaktionsfortschritt mittels DC. Nach beendeter Reaktion wird 2 mal mit 50 ml 0,5 N NaOH gewaschen, getrocknet, eingeengt und der Rückstand aus MTB-Ether/n-Hexan umkristallisiert. Zur weiteren Reinigung wird erneut in Essigester aufgekocht.
Ausbeute 1,6 g (54 % der Theorie), Fp. 208°C.

Analog zu Beispiel I-B-1-c-1 bzw. gemäß den allgemeinen Angaben zur Herstellung wurden folgende Verbindungen der Formel (I-1-c) erhalten:

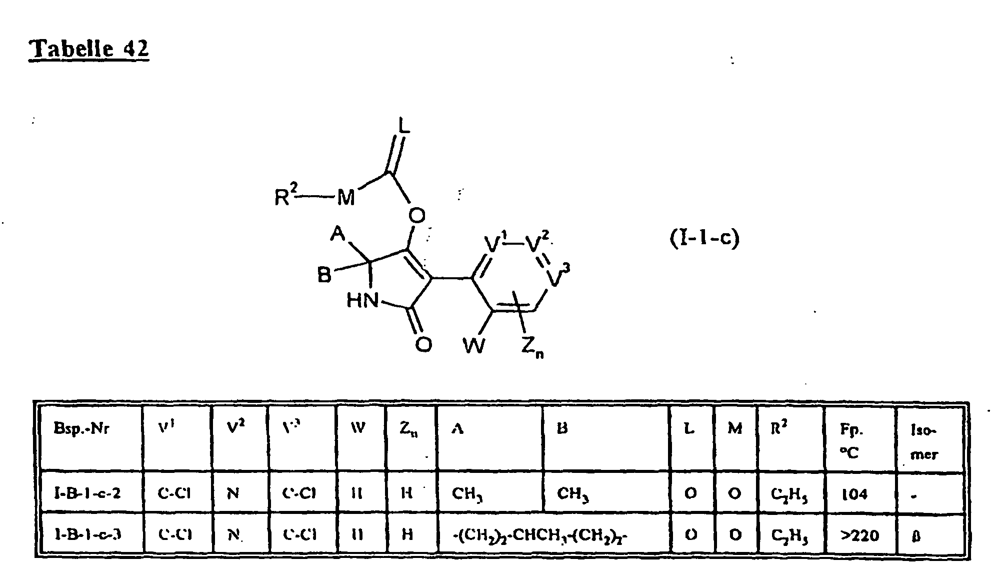

### Beispiel (II-B-1)

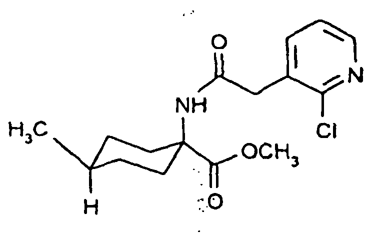

9,5 g cis-4-Methyl-1-amino-cyclohexancarbonsäuremethylesterhydrochlorid und 12,8 ml Triethylamin werden in 90 ml wasserfreiem Tetrahydrofuran (THF) 5 Minuten geruhrt. Nach Zugabe von 7,8 g 3-(2-Chlor)-pyridylessigsäure gemäß Beispiel (XXIII-B-1) wird weitere 15 Minuten bei Raumtemperatur gerührt, dann gibt man 17,8 ml (0,127 mol) Triethylamin zu und tropft sofort 4,3 ml Phosphoroxychlorid so zu, daß die Lösung mäßig siedet. Dann wird 30 Minuten unter Rückfluß erhitzt. Man gießt in 400 ml Eiswasser, extrahiert mit Methylenchlorid, trocknet und engt ein. Der Rückstand wird durch Säulenchromatographie an Kieselgel (Laufmittel Methylenchlorid/Essigsäureethylester 3:1) gereinigt.
Ausbeute 11,3 g (76 % der Theorie), Fp. 124°C.

Analog zu Beispiel II-B-1 bzw. gemäß den allgemeinen Angaben zur Herstellung wurden folgende Verbindungen der Formel (II) erhalten:

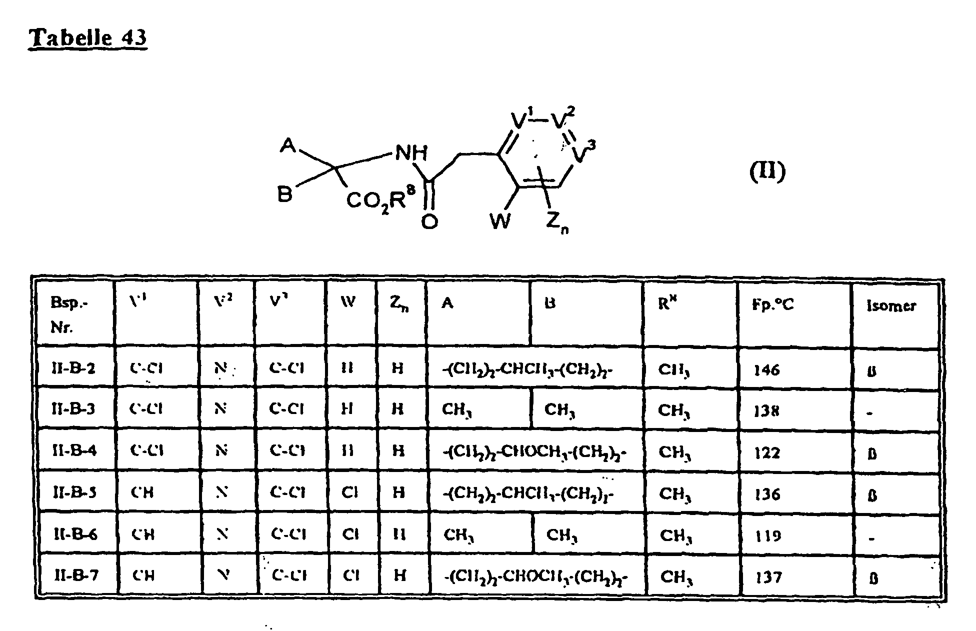

### Beispiel (XXV-B-1)

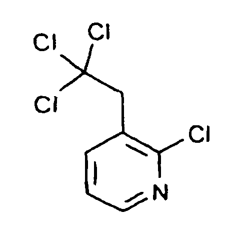

Zu 50 g (0,39 mol) 3-Amino-2-chlorpyridin in 120 ml wasserfreiem Acetonitril tropft man bei Raumtemperatur innerhalb von 5 Minuten 600 g (6,05 mol) 1,1-Dichlorethylen und rührt weitere 5 Minuten. Anschließend gibt man unter Kühlung schnell 62,9 g (0,47 mol) wasserfreies CuCl₂ zu, tropft dann bei Raumtemperatur 70,8 g iso-Pentylnitrit in 240 ml Acetonitril innerhalb von 5 Minuten zu und rührt anschließend bei Raumtemperatur bis zum Ende der Gasentwicklung. Dann gibt man auf 1600 ml eiskalte 20 %ige HCl und extrahiert mehrfach mit Methyl-tert.-butylether (MTB), wäscht die organische Phase mit 20 %iger HCl, trocknet und engt ein.
Ausbeute 165 g. Das Rohprodukt wird ohne weitere Reinigung für die Synthese der Verbindung gemäß Beispiel (XXIV-B-1) eingesetzt.

### Beispiel (XXIV-B-1)

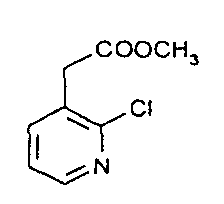

Zu 165 g der rohen Verbindung gemäß Beispiel (XXV-B-1) in 150 ml wasserfreiem Methanol tropft man unter Kühlung 327 ml 30 %ige wäßrige NaHCO₃-Lösung. Man rührt 5 Stunden unter Rückfluß, tropft anschließend bei Raumtemperatur 48 ml konzentrierte Schwefelsäure zu und rührt noch 1 Stunde unter Rückfluß

Man entfernt überschüssiges Methanol im Vakuum, extrahiert den Rückstand mit Methylenchlorid, trocknet und engt ein. Der Rückstand wird destilliert.
Ausbeute 13,1 g (18 % der Theorie), Kp.: 110-115°C bei 0,150 mbar.

### Beispiel (XXIII-B-1)

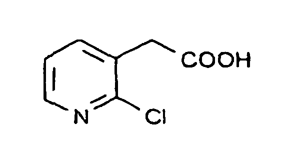

Zu 13,1 g (0,077 mol) der Verbindung gemäß Beispiel (XXIV-B-1) (90 %ig) in 133 ml wasserfreiem THF tropft man bei Raumtemperatur eine Lösung von 4 g (0,095 mol) Lithiumhydroxid (98 %ig) in 133 ml Wasser und rührt einen Tag bei dieser Temperatur. Der Reaktionsfortschritt wird mittels DC verfolgt, gegebenenfalls wird noch mehr Lithiumhydroxid zugesetzt. Dann wird eingeengt, es werden 50 ml Wasser zugegeben, man extrahiert mit MTB-Ether. Danach wird die wäßrige Phase mit 3N HCl angesäuert und nochmal mit MTB-Ether extrahiert. Die wäßrige Phase wird auf pH 4-5 gebracht. Man saugt ab und trocknet.
Ausbeute 7,6 g (51 % der Theorie), Fp.: 197°C.

### Beispiel (XXIII-B-2)

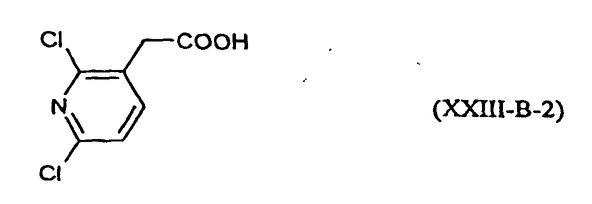

Diese Verbindung wurde nach folgendem Reaktionsschema hergestellt:

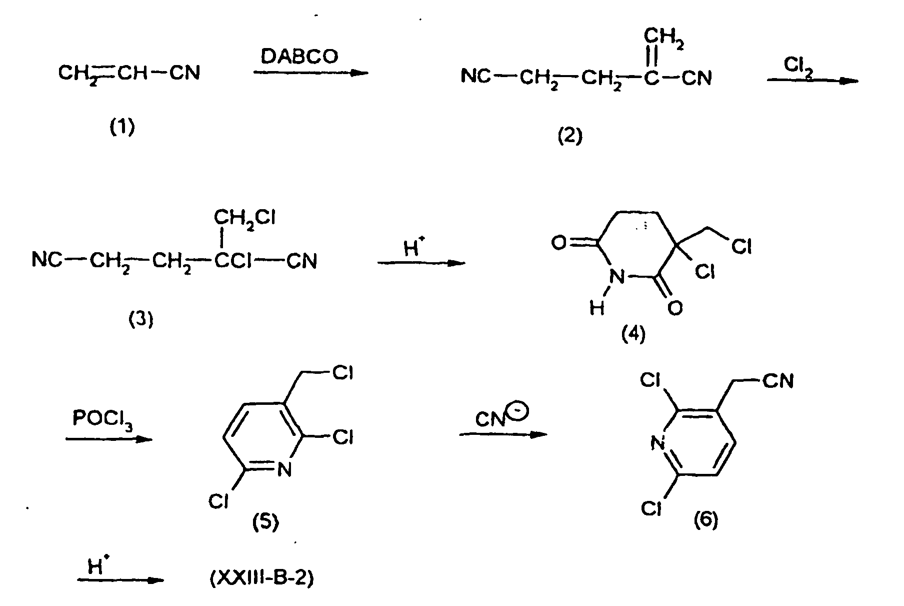

### Verbindung (2)

1060 g Acrylnitril (1) und 1120 g DABCO werden 60 Stunden bei 70°C gerührt. Man läßt abkühlen, gibt 4 l Toluol und 5 l Wasser zu, wäscht die organische Phase mit 1 N HCl, trocknet und engt ein. Der Rückstand wird über einen Dünnschichter bei 0,5 mbar/150°C destilliert.
Ausbeute: 380 g, Kp. 70 bis 72°C/0,20 mbar.

### Verbindung (3)

In das Gemisch von 500 g Verbindung (2), 250 g Tetrachlorkohlenstoff und 5,8 g Benzoylperoxid leitet man bei 70°C 1113 g Chlor ein. Jede Stunde gibt man weitere 5,8 g Benzoylperoxid zu. Anschließend wird eingeengt. Das Rohprodukt wird ohne weitere Reinigung in die Herstellung der Verbindung (4) eingesetzt.
Ausbeute: 862 g (Δ 79 % der Theorie)

### Verbindung (4)

309,1 g Verbindung (3), 950 ml Essigsäure und 221,3 g 78%ige Schwefelsäure werden 2,5 Stunden unter Rückfluß gekocht. Nach dem Abkühlen rührt man das Gemisch in ca. 1800 ml Eiswasser, rührt 1,5 Stunden und saugt ab. Das Rohprodukt wird noch 2 mal mit je 3,5 l Wasser verrührt, abgesaugt, neutral gewaschen und bei 50°C getrocknet.
Ausbeute: 64,3 %, Fp. 131 bis 132°C.

### Verbindung (5)

220 g Verbindung (4) werden unter einem Stickstoffdruck von 5 bar 3 Stunden mit 1,2 l POCl₃ auf 160°C erhitzt. Überschüssiges POCl₃ wird abdestilliert, der Rückstand in Eis gerührt, abgesaugt, gut mit Wasser gewaschen und bei 40°C getrocknet.
Ausbeute: 68 %, NMR (200 MHz, CDCl₃): δ = 4,65 (s,2H, CH₂-Cl), 7,32 (d,1H, Pyrid. 3-H), 7,83 (d,1H, Pyrid. 6-H)

### Verbindung (6)

Zum Gemisch von 31 g (0,64 mol) NaCN und 1 g Aliquat 336 in 50 ml Wasser tropft man bei 60 bis 80°C langsam eine Lösung aus 106 g (0,5 mol) Verbindung (5) (94,5%ig) in 100 ml Toluol und rührt 8 Stunden bei 80°C. Nach Zugabe von 31 g NaCN und 1 g Aliquat 336 rührt man weitere 8 Stunden bei 80°C. Nach dem Abkühlen versetzt man mit je 100 ml Wasser und Toluol, wäscht die organische Phase 2 mal mit je 300 ml gesättigter wässriger NaCl-Lösung, trocknet, engt ein und destilliert im Hochvakuum.
Ausbeute: 29 %, Fp. 80 bis 82°C.

### Beispiel (XXIII-B-2)

Bei 80 bis 90°C gibt man 24,2 g (0,127 mol) Verbindung (6) zu einem Gemisch von 119,3 ml konzentrierter Schwefelsäure und 141,3 ml Wasser und rührt 2 Stunden unter Rückfluß. Nach dem Abkühlen gibt man auf 500 ml Eiswasser, saugt ab, wäscht mit Eiswasser neutral und trocknet.
Ausbeute: 74 %, Fp. 134 bis 135°C.

### Beispiel (XXIII-B-3)

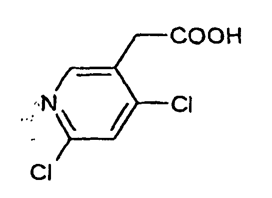

Diese Verbindung wurde nach folgendem Reaktionsschema hergestellt:

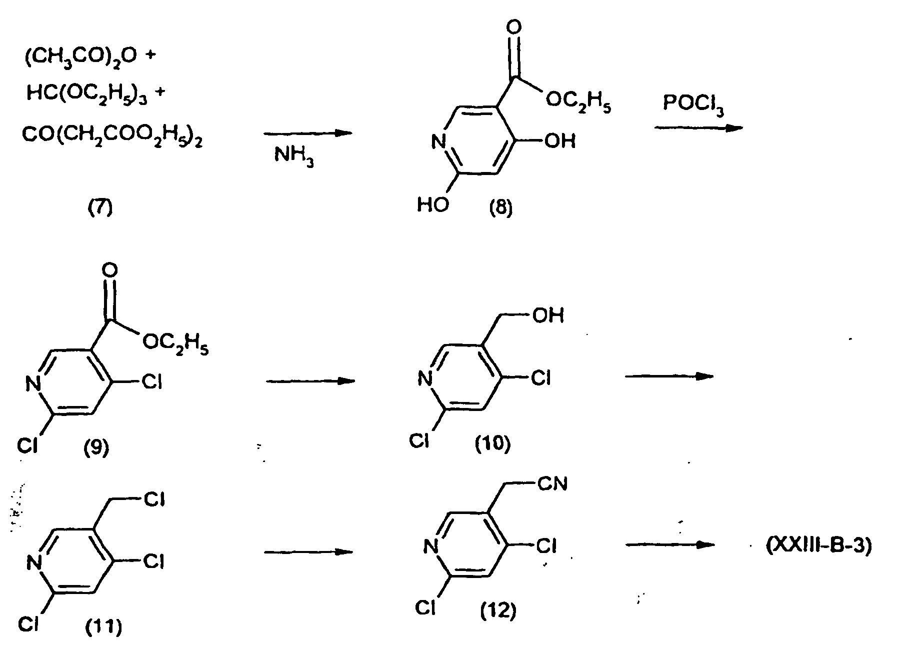

### Verbindung (8)

249 g Acetondicarbonsäurediethylester (7), 233,4 g Acetanhydrid und 164,4 g Triethylorthoformiat werden 1 Stunde bei 140°C gerührt. Man destilliert alle bis 120°C flüchtigen Bestandteile ab und kühlt auf Raumtemperatur ab. Anschließend tropft man 450 ml 25%igen NH₃ zu und saugt den Niederschlag ab. Er wird in Wasser suspendiert. Mit konzentrierter HCl wird pH 1 eingestellt, abgesaugt und über Nacht getrocknet. Das Rohprodukt wird in 1 l Toluol aufgekocht, abgesaugt und getrocknet.
Ausbeute: 107,5 g (51 % der Theorie), Fp. 214°C.

### Verbindung (9)

11,0 g Verbindung (8) werden in 67 ml POCl₃ 7 Stunden unter Rückfluß gekocht. Überschüssiges POCl₃ wird weitgehend abdestilliert und der Rückstand auf Eiswasser gegossen. Durch Zugabe von Na₂CO₃ wird pH 5 eingestellt, mit Methylenchlorid extrahiert, getrocknet, eingeengt und destilliert.
Ausbeute: 8,3 g (63 % der Theorie), Kp. 83°C/0,3 mbar.

### Verbindung (10)

Zu 66,02 g Verbindung (9) in 120 ml trockenem Tetrahydrofuran (THF) gibt man innerhalb von etwa 1 Stunde bei 5 bis 10°C portionsweise 8,6 g Lithiumaluminiumhydrid und rührt noch 2 Stunden bei dieser Temperatur. Dann tropft man bei 5 bis 8°C 250 ml 3N HCl zu und dampft das Reaktionsgemisch Zur Trockne ein. Man extrahiert mit CH₂Cl₂, trocknet, engt ein und reinigt durch Säulenchromatographie mit dem Laufmittel Essigester/Cyclohexan 1/2.
Ausbeute: 32,9 g (61 % der Theorie) Fp. 83°C

### Verbindung (11)

0,94 g Verbindung (10) werden mit 7 ml POCl₃ 19 Stunden unter Rückfluß gekocht. Nach dem Abdestillieren des überschüssigen POCl₃ wird der Rückstand auf Eis gegeben, das Rohprodukt abgesaugt, mit Wasser gewaschen und an der Luft getrocknet.
Ausbeute: 0,25 g (26 % der Theorie), Fp. 53°C.

### Verbindung (12)

Zu 10,27 g Verbindung (11) und 0,9 g Tetrabutylammoniumsulfat in 50 ml Methylenchlorid tropft man bei Raumtemperatur 9,8 g KCN in 38 ml Wasser und rührt über Nacht bei Raumtemperatur. Dann engt man die organische Phase ein und chromatographiert den Rückstand an Kieselgel mit dem Laufmittel Essigester/Cyclohexan 1/2.
Ausbeute: 6,0 g (63 % der Theorie), Fp. 56°C

### (XXIII-B-3)

1 g Verbindung (12) wird in 4,8 ml konzentrierter Schwefelsäure und 5,7 ml Wasser 2 Stunden unter Rückfluß gekocht. Nach dem Abkühlen gibt man auf 20 ml Eiswasser, saugt ab, wäscht neutral und trocknet.
Ausbeute: 0,85 g (81 % der Theorie), Fp. 145°C.

Die Verbindungen gemäß den Beispielen (XXIII-B-1), (XXIII-B-2) und (XXIII-B-3) lassen sich auf allgemein bekannte Art z.B. auch in situ (s. Beispiel II-B-1) in die entsprechenden Säurechloride überführen:

### Beispiel (XX-B-1)

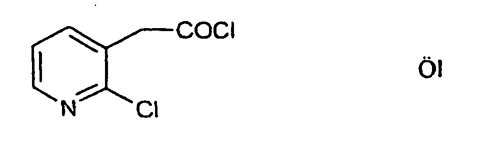

### Beispiel (XX-B-2)

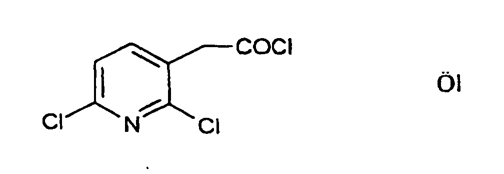

### Beispiel (XX-B-3)

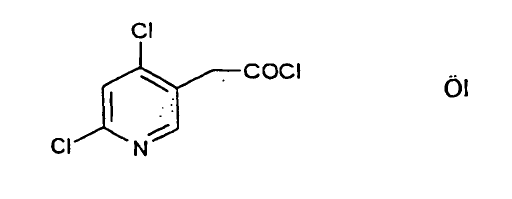

### Anwendungsbeispiele

### Beispiel 1

### Tetranychus-Test (OP-resistent/Spritzbehandlung)

Lösungsmittel:3 GewichtsteileDimethylformamid
Emulgator:1 Gewichtsteil Alkylarylpotygtykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration gespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatte z.B. die Verbindung gemäß Herstellungsbeispiel (I-B-1-a-1) bei einer beispielhaften Wirkstoffkonzentration von 1000 ppm eine Wirkung von 100 % nach 13 Tagen.

### Beispiel 2

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen (I-B-1-a-1), (I-B-1-b-1) und (I-B-1-c-1) bei einer beispielhaften Wirkstoffkonzentration von 0,1 % eine Abtotung von 100 % nach 6 Tagen.

## Patentansprüche

1. Verbindungen der Formel (I) 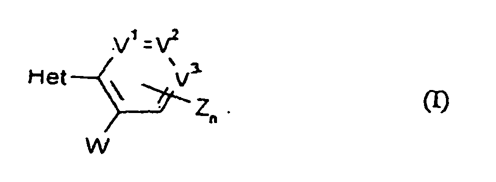 in welcher
A) V¹ für Stickstoff steht und
V² für CH oder C-Z steht und
V³ für CY steht oder
B) V¹ für CX steht und
V² für Stickstoff steht und
V³ für CY steht oder
C) V¹ für CX steht und
V² für CH oder C-Z steht und
V³ für Stickstoff steht
und in welcher
W für Wasserstoff, Nitro, Cyano, Halogen, C₁-C₆-Alkyl, C₂-C₆-Alkenyl, C₂-C₆-Alkinyl, C₁-C₆-Alkoxy, C₁-C₆-Alkylthio, C₁-C₄-Halogenalkyl, C₁-C₄-Halogenalkoxy oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht.
X für Wasserstoff, Halogen, C₁-C₆-Alkyl, C₂-C₆-Alkenyl, C₂-C₆-Alkinyl, C₁-C₆-Alkoxy, C₁-C₆-Alkylthio, C₁-C₄-Halogenalkyl, C₁-C₄-Halogenalkoxy, Cyano, Nitro oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht.
Y für Wasserstoff, Halogen, C₁-C₆-Alkyl, C₁-C₆-Alkoxy, C₁-C₆-Alkylthio, C₁-C₄-Halogenalkyl, C₁-C₄-Halogenalkoxy, Cyano oder Nitro steht.
Z für Halogen, C₁-C₆-Alkyl, C₁-C₆-Alkoxy, C₁-C₆-Alkylthio, C₁-C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht oder
Y und Z gemeinsam für jeweils gegebenenfalls durch Halogen, C₁-C₆-Alkyl, C₁-C₆-Alkoxy oder C₁-C₄-Halogenalkyl substituiertes C₃-C₄-Alkandiyl, C₃-C₄-Alkendiyl oder C₄-Alkandiendiyl stehen, in welchen gegebenenfalls ein bis drei Glieder durch Sauerstoff, Schwefel, Stickstoff oder eine Carbonylgruppe unabhängig voneinander ersetzt sein können, wobei in diesem Fall n für 1 steht, oder
W und Z gemeinsam für jeweils gegebenenfalls durch Halogen, C₁-C₆-Alkyl, C₁-C₆-Alkoxy oder C₁-C₄-Halogenalkyl substituiertes C₃-C₄-Alkandiyl, C₃-C₄-Alkendiyl oder C₄-Alkandiendiyl, stehen, in welchen gegebenenfalls ein bis drei Glieder durch Sauerstoff, Schwefel, Stickstoff oder eine Carbonylgruppe unabhängig voneinander ersetzt sein können, wobei in diesem Fall n für 1 steht.
n für 0, 1 oder 2 steht, wobei für n = 2 die Substituenten Z gleich oder verschieden sein können.
Het für die Gruppe 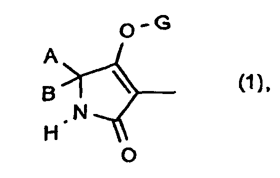 steht
A für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff steht.
B für Wasserstoff, C₁-C₁₂-Alkyl oder C₁-C₈-Alkoxy-C₁-C₆-alkyl steht oder
A, B und das Kohlenstoffatom an das sie gebunden sind, für C₃-C₁₀-Cycloalkyl oder C₅-C₁₀-Cycloalkenyl stehen, worin jeweils gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
A, B und das Kohlenstoffatom, an das sie gebunden sind, für C₅-C₆-Cycloalkyl stehen, welches durch eine gegebenenfalls ein oder zwei Sauerstoff- und/oder Schwefelatome enthaltende Alkylendiyl-, oder durch eine Alkylendioxy- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind für C₃-C₈-Cycloalkyl oder C₅-C₈-Cycloalkenyl stehen, worin zwei Kohlenstoffatome durch jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl miteinander verbunden sind, worin jeweils gegebenenfalls eine Methylengruppe durch Sauerstoff oder Schwefel ersetzt ist.
G für Wasserstoff (a) oder für eine der Gruppen 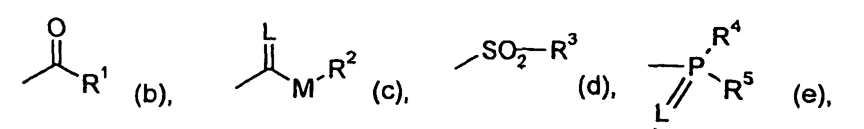 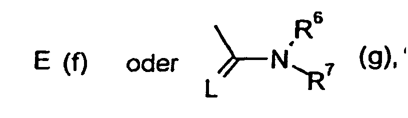 steht
in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht mit ein oder zwei Heteroatomen aus der Reihe Sauerstoff, Schwefel und Stickstoff.
R² für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht.
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht.
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen.
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

2. Verfahren zur Herstellung von Verbindungen der Formel (I) gemäß Anspruch 1, **dadurch gekennzeichnet, daß** man
(A) Verbindungen der Formel (I-1-a) 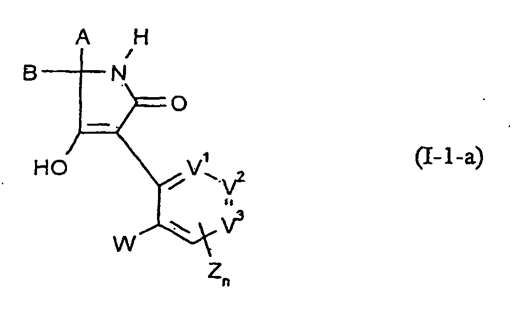 in welcher
A, B, W, V¹, V², V³, n und Z die in Anspruch 1 angegebenen Bedeutungen haben,
erhält, wenn man
Verbindungen der Formel (II) 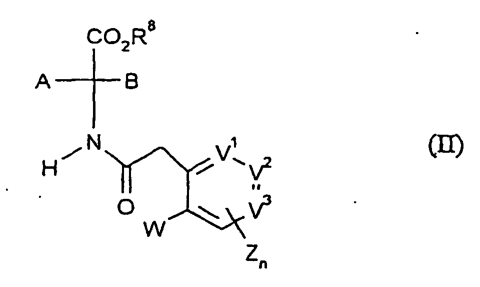 in welcher
A, B, W, V¹, V², V³, n und Z die in Anspruch 1 angegebenen Bedeutungen haben,
und
R⁸ für Alkyl steht,
in Gegenwart eines Verdünnungsmittels und in Gegenwart einer Base intramolekular kondensiert, und gegebenenfalls anschließend die so erhaltenen Verbindungen der Formeln (I-1-a) jeweils
(F)
α) mit Säurehalogeniden der Formel (VIII) 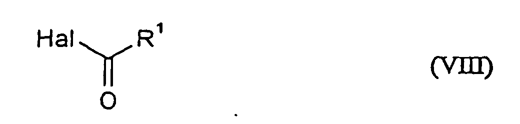 in welcher
R¹ die in Anspruch 1 angegebene Bedeutung hat und
Hal für Halogen steht
oder
β) mit Carbonsäureanhydriden der Formel (IX)
R¹-CO-O-CO-R¹ (IX)
in welcher
R¹ die oben angegebene Bedeutung hat,
gegebenenfalls in Gegenwart eines Verdünnungsmittels und gegebenenfalls in Gegenwart eines Säurebindemittels umsetzt
oder
(G) mit Chlorameisensäureestern oder Chlorameisensaurethioestern der Formel (X)
R²-M-CO-Cl (X)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt
oder
(H)
α) mit Chlormonothioameisensäureestern oder Chlordithioameisensäureestern der Formel (XI) 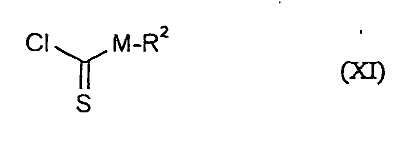 in welcher
M und R² die oben angegebenen Bedeutungen haben,
gegebenenfalls in Gegenwart eines Verdünnungsmittels und gegebenenfalls in Gegenwart eines Säurebindemittels umsetzt
oder
β) mit Schwefelkohlenstoff und anschließend mit Verbindungen der Formel (XII)
R²-Hal (XII)
in welcher
R² die oben angegebene Bedeutung hat und
Hal für Chlor, Brom oder Iod steht,
gegebenenfalls in Gegenwart eines Verdünnungsmittels und gegebenenfalls in Gegenwart einer Base umsetzt
oder
(I) mit Sulfonsäurechloriden der Formel (XIII)
R³-SO₂-Cl (XIII)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt
oder
(J) mit Phosphorverbindungen der Formel (XIV) 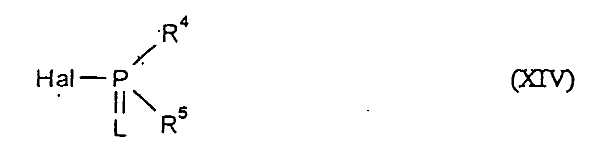 in welcher
L, R⁴ und R⁵ die in Anspruch 1 angegebenen Bedeutungen haben und
Hal für Halogen steht,
gegebenenfalls in Gegenwart eines Verdünnungsmittels und gegebenenfalls in Gegenwart eines Säurebindemittels umsetzt
oder
(K) mit Metallverbindungen oder Aminen der Formeln (XV) oder (XVI)
Me(OR¹⁰)ₜ (XV) 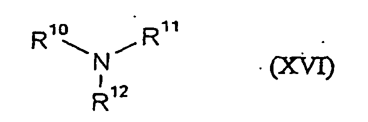
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt
oder
(L)
α) mit Isocyanaten oder Isothiocyanaten der Formel (XVII)
R⁶-N=C=L (XVII)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt
oder
β) mit Carbamidsäurechloriden oder Thiocarbamidsäurechloriden der Formel (XVIII) 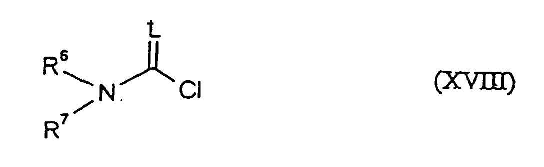 in welcher
L, R⁶ und R⁷ die in Anspruch 1 angegebenen Bedeutungen haben,
gegebenenfalls in Gegenwart eines Verdünnungsmittels und gegebenenfalls in Gegenwart eines Säurebindemittels, umsetzt.

3. Verbindungen der Formel (II) 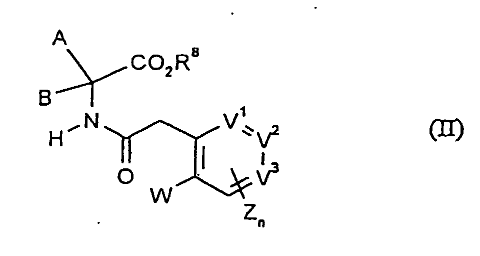 in welcher
W, X, V¹, V², V³ und n die in Anspruch 1 angegebenen Bedeutungen haben,
R⁸ für Alkyl steht,
sind
A für jeweils gegebenenfalls durch Halogen substituiertes Alkyl, Alkenyl, Alkoxyalkyl, Polyalkoxyalkyl oder Alkylthioalkyl, für jeweils gesättigtes oder ungesättigtes und gegebenenfalls substituiertes Cycloalkyl oder Heterocyclyl oder für jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Alkyl oder Alkoxyalkyl steht, oder
A und B gemeinsam mit dem Kohlenstoffatom an das sie gebunden sind für einen gesättigten oder ungesättigten, gegebenenfalls substituierten Carbocyclus oder Heterocyclus stehen.

4. Schädlingsbekämpfungsmittel und Herbizide, **gekennzeichnet durch** einen Gehalt an mindestens einer Verbindung der Formel (I) gemäß Anspruch 1.

5. Verwendung von Verbindungen der Formel (I) gemäß Anspruch 1 zur Bekämpfung von Schädlingen und Unkräutern.

6. Verfahren zur Bekämpfung von Schädlingen und Unkräutern, **dadurch gekennzeichnet, daß** man Verbindungen der Formel (I) gemäß Anspruch 1 auf Schädlinge bzw. Unkräutern und/oder ihren Lebensraum einwirken läßt.

7. Verfahren zur Herstellung von Schädlingsbekämpfungsmitteln und herbiziden Mitteln, **dadurch gekennzeichnet, daß** man Verbindungen der Formel (I) gemäß Anspruch 1 mit Streckmitteln und/oder oberflächenaktiven Mitteln vermischt.

## Claims

1. Compounds of the formula (I) 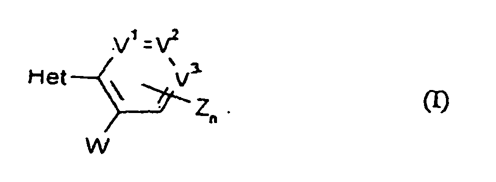 in which
A) V¹ represents nitrogen and
V² represents CH or C-Z and
V³ represents CY or
B) V¹ represents CX and
V² represents nitrogen and
V³ represents CY or
C) V¹ represents CX and
V² represents CH or C-Z and
V³ represents nitrogen
and in which
W represents hydrogen, nitro, cyano, halogen, C₁-C₆-alkyl, C₂-C₆-alkenyl, C₂-C₆-alkinyl, C₁-C₆-alkoxy, C₁-C₆-alkylthio, C₁-C₄-halogenoalkyl, C₁-C₄-halogenoalkoxy, or represents phenyl, phenoxy, phenylthio, phenyl-C₁-C₄-alkoxy or phenyl-C₁-C₄-alkylthio, each of which is optionally substituted by halogen, C₁-C₆-alkyl, C₁-C₆-alkoxy, C₁-C₄-halogenoalkyl, C₁-C₄-halogenoalkoxy, nitro or cyano.
X represents hydrogen, halogen, C₁-C₆-alkyl, C₂-C₆-alkenyl, C₂-C₆-alkinyl, C₁-C₆-alkoxy, C₁-C₆-alkylthio, C₁-C₄-halogenoalkyl, C₁-C₄-halogenoalkoxy, cyano, nitro, or represents phenyl, phenoxy, phenylthio, phenyl-C₁-C₄-alkoxy or phenyl-C₁-C₄-alkylthio, each of which is optionally substituted by halogen, C₁-C₆-alkyl, C₁-C₆-alkoxy, C₁-C₄-halogenoalkyl, C₁-C₄-halogenoalkoxy, nitro or cyano.
Y represents hydrogen, halogen, C₁-C₆-alkyl, C₁-C₆-alkoxy, C₁-C₆-alkylthio, C₁-C₄-halogenoalkyl, C₁-C₄-halogenoalkoxy, cyano or nitro.
Z represents halogen, C₁-C₆-alkyl, C₁-C₆-alkoxy, C₁-C₆-alkylthio, C₁-C₄-halogenoalkyl, C₁-C₄-halogenoalkoxy, hydroxyl, cyano, nitro, or represents phenoxy, phenylthio, thiazolyloxy, pyridinyloxy, pyrimidyloxy, pyrazolyloxy, phenyl-C₁-C₄-alkoxy or phenyl-C₁-C₄-alkylthio, each of which is optionally substituted by halogen, C₁-C₄-alkyl, C₁-C₄-alkoxy, C₁-C₄-halogenoalkyl, C₁-C₄-halogenoalkoxy, nitro or cyano, or
Y and Z together represent C₃-C₄-alkanediyl, C₃-C₄-alkenediyl or C₄-alkanedienediyl which are in each case optionally substituted by halogen, C₁-C₆-alkyl, C₁-C₆-alkoxy or C₁-C₄-halogenoalkyl and in which one to three members independently of one another can optionally be replaced by oxygen, sulphur, nitrogen or a carbonyl group, in which case n represents 1, or
W and Z together represent C₃-C₄-alkanediyl, C₃-C₄-alkenediyl or C₄-alkanedienediyl which are in each case optionally substituted by halogen, C₁-C₆-alkyl, C₁-C₆-alkoxy or C₁-C₄-halogenoalkyl and in which one to three members independently of one another can optionally be replaced by oxygen, sulphur, nitrogen or a carbonyl group, in which case n represents 1.
n represents 0, 1 or 2, it being possible for the substituents Z to be identical or different if n = 2.
Het represents the group 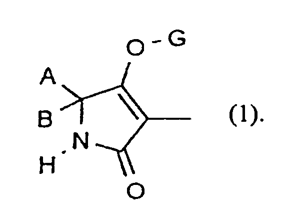
A represents hydrogen, or represents C₁-C₁₂-alkyl, C₂-C₈-alkenyl, C₁-C₁₀-alkoxy-C₁-C₈-alkyl, poly-C₁-C₈-alkoxy-C₁-C₈-alkyl or C₁-C₁₀-alkylthio-C₁-C₆-alkyl, each of which is optionally substituted by halogen, or represents C₃-C₈-cycloalkyl which is optionally substituted by halogen, C₁-C₆-alkyl or C₁-C₆-alkoxy and in which one or two methylene groups which are not directly adjacent are optionally replaced by oxygen and/or sulphur, or represents phenyl, naphthyl, phenyl-C₁-C₆-alkyl, naphthyl-C₁-C₆-alkyl or hetaryl having 5 or 6 ring atoms and one to three hetero atoms from the series consisting of oxygen, sulphur and nitrogen, each of which is optionally substituted by halogen, C₁-C₆-alkyl, C₁-C₆-halogenoalkyl, C₁-C₆-alkoxy, C₁-C₆-halogenoalkoxy, cyano or nitro.
B represents hydrogen, C₁-C₁₂-alkyl or C₁-C₈-alkoxy-C₁-C₆-alkyl, or
A, B and the carbon atom to which they are bonded represent C₃-C₁₀-cycloalkyl or C₅-C₁₀-cycloalkenyl, in each of which one methylene group is optionally replaced by oxygen or sulphur and which are optionally substituted by C₁-C₈-alkyl, C₃-C₁₀-cycloalkyl, C₁-C₈-halogenoalkyl, C₁-C₈-alkoxy, C₁-C₈-alkylthio, halogen or phenyl, or
A, B and the carbon atom to which they are bonded represent C₅-C₆-cycloalkyl which is substituted by an alkylenediyl group which optionally contains one or two oxygen and/or sulphur atoms or by an alkylenedioxy group or by an alkylenedithioyl group, this group together with the carbon atom to which it is bonded forming a further five- to eight-membered ring, or
A, B and the carbon atom to which they are bonded represent C₃-C₈-cycloalkyl or C₅-C₈-cycloalkenyl, in which two carbon atoms are joined together by C₃-C₆-alkanediyl, C₃-C₆-alkenediyl or C₄-C₆-alkanedienediyl, each of which is optionally substituted by C₁-C₆-alkyl, C₁-C₆-alkoxy or halogen and in each of which one methylene group is optionally replaced by oxygen or sulphur.
G represents hydrogen (a) or one of the groups 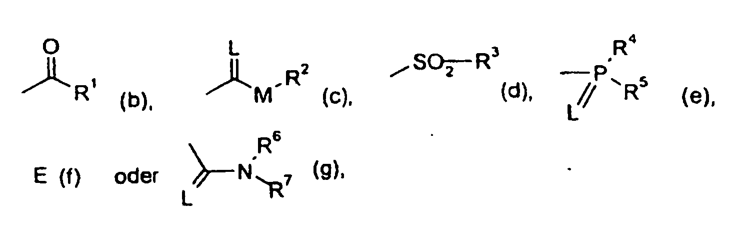 in which
E represents a metal ion equivalent or an ammonium ion,
L represents oxygen or sulphur and
M represents oxygen or sulphur.
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl, each of which is optionally substituted by halogen, or represents C₃-C₈-cycloalkyl which is optionally substituted by halogen, C₁-C₆-alkyl or C₁-C₆-alkoxy and in which one or two methylene groups which are not directly adjacent are optionally replaced by oxygen and/or sulphur,
or represents phenyl which is optionally substituted by halogen, cyano, nitro, C₁-C₆-alkyl, C₁-C₆-alkoxy, C₁-C₆-halogenoalkyl, C₁-C₆-halogenoalkoxy, C₁-C₆-alkylthio or C₁-C₆-alkylsulphonyl,
or represents phenyl-C₁-C₆-alkyl which is optionally substituted by halogen, nitro, cyano, C₁-C₆-alkyl, C₁-C₆-alkoxy, C₁-C₆-halogenoalkyl or C₁-C₆-halogenoalkoxy,
or represents 5- or 6-membered hetaryl having one or two hetero atoms from the series consisting of oxygen, sulphur or nitrogen which is optionally substituted by halogen or C₁-C₆-alkyl,
or represents phenoxy-C₁-C₆-alkyl which is optionally substituted by halogen or C₁-C₆-alkyl,
or represents 5- or 6-membered hetaryloxy-C₁-C₆-alkyl having one or two hetero atoms from the series consisting of oxygen, sulphur and nitrogen which is optionally substituted by halogen, amino or C₁-C₆-alkyl.
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl, each of which is optionally substituted by halogen,
or represents C₃-C₈-cycloalkyl which is optionally substituted by halogen, C₁-C₆-alkyl or C₁-C₆-alkoxy,
or represents phenyl or benzyl, each of which is optionally substituted by halogen, cyano, nitro, C₁-C₆-alkyl, C₁-C₆-alkoxy, C₁-C₆-halogenoalkyl or C₁-C₆-halogenoalkoxy.
R³ represents C₁-C₈-alkyl which is optionally substituted by halogen, or represents phenyl or benzyl, each of which is optionally substituted by halogen, C₁-C₆-alkyl, C₁-C₆-alkoxy, C₁-C₄-halogenoalkyl, C₁-C₄-halogenoalkoxy, cyano or nitro.
R⁴ and R⁵ independently of one another represent C₁-C₈-alkyl, C₁-C₈-alkoxy, C₁-C₈-alkylamino, di-(C₁-C₈-alkyl)amino, C₁-C₈-alkylthio or C₃-C₈-alkenylthio, each of which is optionally substituted by halogen, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl.
R⁶ and R⁷ independently of one another represent hydrogen, or represent C₁-C₈=-alkyl, C₃-C₈-cycloalkyl, C₁-C₈-alkoxy, C₃-C₈-alkenyl or C₁-C₈-alkoxy-C₂-C₈-alkyl, each of which is optionally substituted by halogen, or represent phenyl or benzyl, each of which is optionally substituted by halogen, C₁-C₈-alkyl, C₁-C₈-halogenoalkyl or C₁-C₈-alkoxy, or together represent a C₃-C₆-alkylene radical in which one methylene group is optionally replaced by oxygen or sulphur and which is optionally substituted by C₁-C₆-alkyl.

2. Process for the preparation of compounds of the formula (I) according to Claim 1, **characterized in that**
(A) Compounds of the formula (I-1-a) 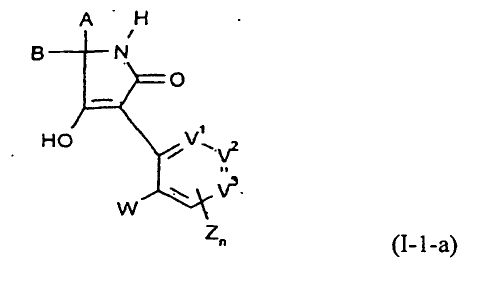 in which
A, B, W, V¹, V², V³, n and Z have the meanings given in Claim 1
are obtained when
compounds of the formula (II) 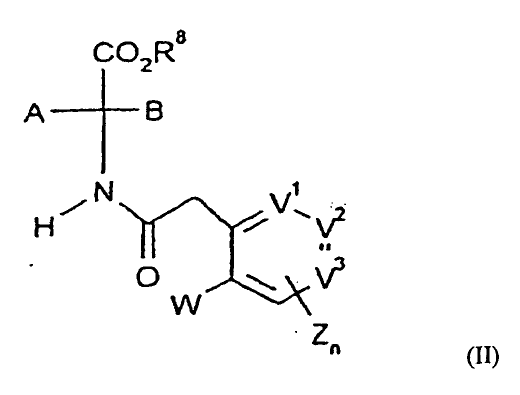 in which
A, B, W, V¹, V², V³, n and Z have the meanings given in Claim 1
and
R⁸ represents alkyl
are subjected to an intramolecular condensation reaction in the presence of a diluent and in the presence of a base,
and, if appropriate, subsequently the resulting compounds of the formula (I-1-a) are reacted in each case
(F)
α) with acid halides of the formula (VIII) 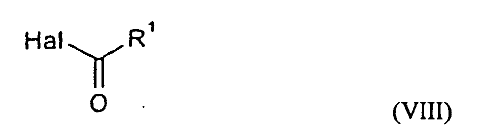 in which
R¹ has the meaning given in Claim 1 and
Hal represents halogen,
or
β) with carboxylic anhydrides of the formula (IX)
R¹-CO-O-CO-R¹ (IX)
in which
R¹ has the abovementioned meaning,
if appropriate in the presence of a diluent and if appropriate in the presence of an acid-binding agent,
or
(G) with chloroformic esters or chloroformic thioesters of the formula (X)
R²-M-CO-Cl (X)
in which
R² and M have the meanings given in Claim 1,
if appropriate in the presence of a diluent and if appropriate in the presence of an acid-binding agent,
or
(H)
α) with chloromonothioformic esters or chlorodithioformic esters of the formula (XI) 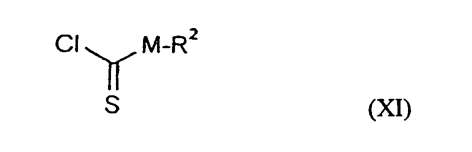 in which
M and R² have the abovementioned meanings,
if appropriate in the presence of a diluent and if appropriate in the presence of an acid-binding agent,
or
β) with carbon disulphide and subsequently with compounds of the formula (XII)
R²-Hal (XII)
in which
R² has the abovementioned meaning and
Hal represents chlorine, bromine or iodine,
if appropriate in the presence of a diluent and if appropriate in the presence of a base,
or
(I) with sulphonyl chlorides of the formula (XIII)
R³-SO₂-Cl (XIII)
in which
R³ has the meaning given in Claim 1,
if appropriate in the presence of a diluent and if appropriate in the presence of an acid-binding agent,
or
(J) with phosphorus compounds of the formula (XIV) 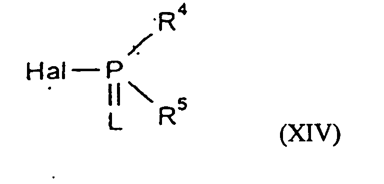 in which
L, R⁴ and R⁵ have the meanings given in Claim 1 and
Hal represents halogen,
if appropriate in the presence of a diluent and if appropriate in the presence of an acid-binding agent,
or
(K) with metal compounds or amines of the formulae (XV) or (XVI)
Me(OR¹⁰)ₜ (XV) 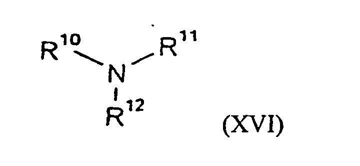
in which
Me represents a mono- or divalent metal,
t represents the number 1 or 2 and
R¹⁰, R¹¹, R¹² independently of one another represent hydrogen or alkyl,
if appropriate in the presence of a diluent,
or
(L)
α) with isocyanates or isothiocyanates of the formula (XVII)
R⁶-N=C=L (XVII)
in which
R⁶ and L have the meanings given in Claim 1,
if appropriate in the presence of a diluent and if appropriate in the presence of a catalyst,
or
β) with carbamoyl chlorides or thiocarbamoyl chlorides of the formula (XVIII) 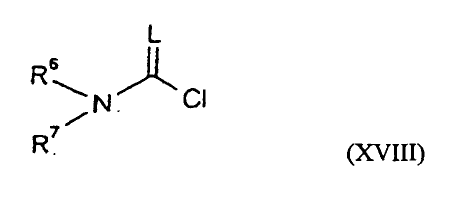 in which
L, R⁶ and R⁷ have the meanings given in Claim 1,
if appropriate in the presence of a diluent and if appropriate in the presence of an acid-binding agent.

3. Compounds of the formula (II) 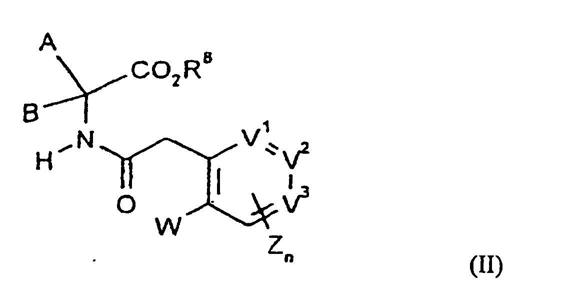 in which
W, X, V¹, V², V³ and n have the meanings given in Claim 1,
R⁸ represents alkyl and
A represents alkyl, alkenyl, alkoxyalkyl, polyalkoxyalkyl or alkylthioalkyl, each of which is optionally substituted by halogen, or represents in each case saturated or unsaturated and optionally substituted cycloalkyl or heterocyclyl, or represents aryl, arylalkyl or hetaryl, each of which is optionally substituted by halogen, alkyl, halogenoalkyl, alkoxy, halogenoalkoxy, cyano or nitro,
B represents alkyl or alkoxyalkyl, or
A and B together with the carbon atom to which they are bonded represent a saturated or unsaturated, optionally substituted carbocycle or heterocycle.

4. Pesticides and herbicides, **characterized in that** they comprise at least one compound of the formula (I) according to Claim 1.

5. Use of compounds of the formula (I) according to Claim 1 for controlling pests and weeds.

6. Method of controlling pests and weeds, **characterized in that** compounds of the formula (I) according to Claim 1 are allowed to act on pests and weeds, respectively, and/or their environment.

7. Process for the preparation of pesticides and herbicidal compositions, **characterized in that** compounds of the formula (I) according to Claim 1 are mixed with extenders and/or surfactants.

## Revendications

1. Composés de formule (I) 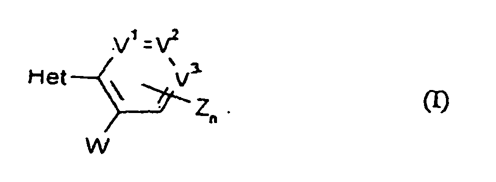 dans laquelle
A) V1 représente l'azote et
V2 représente un groupe CH ou C-Z et
V3 représente un groupe CY, ou bien
B) V1 représente un groupe CX et
V2 représente l'azote et
V3 représente un groupe CY, ou bien
C) V1 représente un groupe CX et
V2 représente un groupe CH ou C-Z et
V3 représente l'azote
et dans laquelle
W représente l'hydrogène, un groupe nitro, cyano, un halogène, un reste alkyle en C₁ à C₆, alcényle en C₂ à C₆, alcinyle en C₂ à C₆, alkoxy en C₁ à C₆, alkylthio en C₁ à C₆, halogénalkyle en C₁ à C₄, halogénalkoxy en C₁ à C₄ ou un reste phényle, phénoxy, phénylthio, phényl-(alkoxy en C₁ à C₄) ou phényl-(alkylthio en C₁ à C₄), chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
X représente l'hydrogène, un halogène, un reste alkyle en C₁ à C₆, alcényle en C₂ à C₆, alcinyle en C₂ à C₆, alkoxy en C₁ à C₆, alkylthio en C₁ à C₆, halogénalkyle en C₁ à C₄, halogénalkoxy en C₁ à C₄, cyano, nitro ou un reste phényle, phénoxy, phénylthio, phényl-(alkoxy en C₁ à C₄) ou phényl- (alkylthio en C₁ à C₄), chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
Y représente l'hydrogène, un halogène, un reste alkyle en C₁ à C₆, alkoxy en C₁ à C₆, alkylthio en C₁ à C₆, halogénalkyle en C₁ à C₄, halogénalkoxy en C₁ à C₄, cyano ou nitro,
Z représente un halogène, un reste alkyle en C₁ à C₆, alkoxy en C₁ à C₆, alkylthio en C₁ à C₆, halogénalkyle en C₁ à C₄, halogénalkoxy en C₁ à C₄, hydroxy, cyano, nitro, ou un reste phénoxy, phényltio, thiazolyloxy, pyridinyloxy, pyrimidyloxy, pyrazolyloxy, phényl-(alkyloxy en C₁ à C₄) ou phényl-(alkylthio en C₁ à C₄), chacun éventuellement substitué par un radical halogéno, alkyle en C₁ à C₄, alkoxy en C₁ à C₄, halogénalkyle en C₁ à C₄, halogénalkoxy en C₁ à C₄, nitro ou cyano, ou bien
Y et Z forment ensemble un reste alcanediyl en C₃ ou C₄, alcènediyl en C₃ ou C₄ ou alcanediendiyl en C₄, chacun éventuellement substitué par un radical halogéno, alkyle en C₁ à C₆, alkoxy en C₁ à C₆ ou halogénalkyle en C₁ à C₄ et dans lesquels, le cas échéant, un à trois chaînons peuvent être remplacés indépendamment les uns des autres par de l'oxygène, du soufre, de l'azote ou un groupe carbonyle, auquel cas n est égal à 1, ou bien
W et Z forment ensemble un reste alcanediyl en C3 ou C4, alcènediyl en C3 ou C4 ou alcanediendiyl en C4, chacun éventuellement substitué par un radical halogéno, alkyle en C1 à C6, alkoxy en C1 à C6 ou halogénalkyle en C1 à C4 et dans lesquels, le cas échéant, un à trois chaînons peuvent être remplacés indépendamment les uns des autres par de l'oxygène, du soufre, de l'azote ou un groupe carbonyle, auquel cas n est égal à 1,
n a la valeur 0, 1 ou 2, les substituants Z pouvant être égaux ou différents au cas où n est égal à 2,
Het représente le groupe 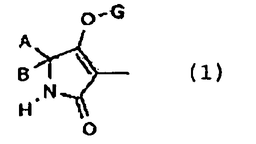
A représente l'hydrogène, un reste alkyle en C₁ à C₁₂, alcényle en C₂ à C₈, (alkoxy en C₁ à C₁₀)-(alkyle en C₁ à C₈), poly(alkoxy en C₁ à C₈)-(alkyle en C₁ à C₈) ou (alkylthio en C₁ à C₁₀)-(alkyle en C₁ à C₆), chacun éventuellement substitué par un halogène, un reste cycloalkyle en C₃ à C₆, éventuellement substitué par un radical halogéno ou alkyle en C₁ à C₆ ou alkoxy en C₁ à C₆ et dans lequel, le cas échéant, un ou deux groupes méthylène non directement voisins sont remplacés par de l'oxygène et/ou du soufre, ou bien un reste phényle, naphtyle, phényl-(alkyle en C₁ à C₆), naphtyl-(alkyle en C₁ à C₆) ou hétaryle à noyau de 5 ou 6 atomes ayant un à trois hétéroatomes de la série oxygène, soufre et azote, chaque reste étant éventuellement substitué par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un reste alkyle en C₁ à C₁₂ ou (alkoxy en C₁ à C₈)-(alkyle en C₁ à C₆), ou bien
A, B et l'atome d'azote auquel ils sont liés représentent un reste cycloalkyle en C₃ à C₁₀ ou cycloalcényle en C₅ à C₁₀, dans chacun desquels, le cas échéant, un groupe méthylène est remplacé par de l'oxygène ou du soufre et qui sont éventuellement substitués par un radical alkyle en C₁ à C₈, cycloalkyle en C₃ à C₁₀, halogénalkyle en C₁ à C₈, alkoxy en C₁ à C₈, alkylthio en C₁ à C₈, halogéno ou phényle, ou bien
A, B et l'atome de carbone auquel ils sont liés représentent un reste cycloalkyle en C₅ ou C₆ qui est éventuellement substitué par un radical alkylènediyl contenant éventuellement un ou deux atomes d'oxygène et/ou de soufre, ou par un groupe alkylènedioxy ou par un groupe alkylènedithioyl qui forme un autre noyau pentagonal à octogonal avec l'atome de carbone auquel il est lié, ou bien
A, B et l'atome de carbone auquel ils sont liés forment un reste cycloalkyle en C₃ à C₈ ou cycloalcényle en C₅ à C₈, dans lesquels deux atomes de carbone sont liés ensemble par un radical alcanediyl en C₃ à C₆, alcènediyl en C₃ à C₆ ou alcanediendiyl en C₄ à C₆, chacun éventuellement substitué par un radical alkyle en C₁ à C₆, alkoxy en C₁ à C₆ ou halogéno, et dans chacun desquels, le cas échéant, un groupe méthylène est remplacé par de l'oxygène ou du soufre,
G représente l'hydrogène (a) ou l'un des groupes 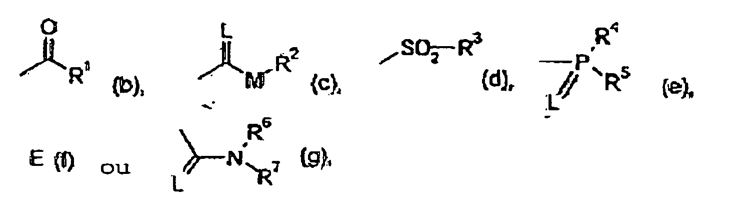 dans lesquels
E représente un équivalent d'ions métalliques ou un ion ammonium,
L représente l'oxygène ou le soufre et
M représente l'oxygène ou le soufre,
R¹ est un reste alkyle en C₁ à C₂₀, alcényle en C₂ à C₂₀, (alkoxy en C₁ à C₈)-(alkyle en C₁ à C₈), (alkylthio en C₁ à C₈)-(alkyle en C₁ à C₈) ou poly (alkoxy en C₁ à C₈)-(alkyle en C₁ à C₈), chacun éventuellement substitué par un halogène, ou un reste cycloalkyle en C₃ à C₈ éventuellement substitué par un radical halogéno, alkyle en C₁ à C₆ ou alkoxy en C₁ à C₆, et dans lequel, le cas échéant, un ou deux groupes méthylène non directement voisins sont remplacés par de l'oxygène et/ou par du soufre,
un reste phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyl en C₁ à C₆,
un reste phényl-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆,
un reste hétaryle pentagonal ou hexagonal ayant un ou deux hétéroatomes de la série oxygène, soufre et azote et éventuellement substitué par un radical halogéno ou alkyle en C₁ à C₆,
un reste phénoxy-(alkyle en C₁ à C₆) éventuellement substitué par un radical halogéno ou alkyle en C₁ à C₆, ou bien
un reste hétaryloxy-(alkyle en C₁ à C₆) ayant un ou deux hétéroatomes de la série oxygène, soufre et azote et éventuellement substitué par un radical halogéno, amino ou alkyle en C₁ à C₅,
R² représente un reste alkyle en C₁ à C₂₀, alcényle en C₂ à C₂₀, (alkoxy en C₁ à C₈)-(alkyle en C₂ à C₈) ou poly-(alkoxy en C₁ à C₈) - (alkyle en C₂ à C₈) chacun éventuellement substitué par un halogène,
un reste cycloalkyle en C₃ à C₈ éventuellement substitué par un radical halogéno ou alkyle en C₁ à C₆ ou alkoxy en C₁ à C₆, ou bien
un reste phényle ou benzyle, chacun éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène, ou un reste phényle ou benzyle, chacun éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent indépendamment l'un de l'autre un reste alkyle en C₁ à C₈, alkoxy en C₁ à C₈, alkylamino en C₁ à C₈, di(alkyle en C₁ à C₈) amino, alkylthio en C₁ à C₈ ou alcénylthio en C₃ à C₈, chacun éventuellement substitué par un halogène, ou un reste phényle, phénoxy ou phénylthio, chacun éventuellement substitué par un radical halogéno, nitro, cyano, alkoxy en C₁ à C₄, halogénalkoxy en C1 à C₄, alkylthio en C₁ à C₄, halogénalkylthio en C₁ à C₄, alkyle en C₁ à C₄ ou halogénalkyle en C₁ à C₄,
R⁶ et R⁷ représentent indépendamment l'un de l'autre l'hydrogène, un reste alkyle en C₁ à C₈, cycloalkyle en C₃ à C₈, alkoxy en C₁ à C₈, alcényle en C₃ à C₈ ou (alkoxy en C₁ à C₈)-(alkyle en C₂ à C₈), chacun éventuellement substitué par un halogène, un reste phényle ou benzyle, chacun éventuellement substitué par un radical halogéno, alkyle en C₁ à C₈, halogénalkyle en C₁ à C₈ ou alkoxy en C₁ à C₈, ou forment ensemble un reste alkylène en C₃ à C₆ éventuellement substitué par un radical alkyle en C₁ à C₆ et dans lequel, le cas échéant, un groupe méthylène est remplacé par de l'oxygène ou du soufre.

2. Procédé de production de composés de formule (I) suivant la revendication 1, **caractérisé en ce que**
(A) on obtient des composés de formule (I-1-a) 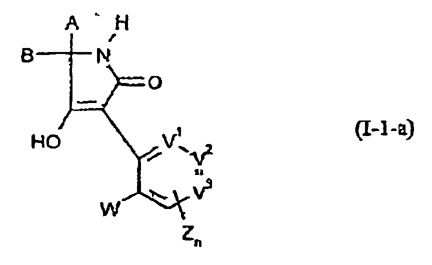 dans laquelle
A, B, W, V¹, V², V³, n et Z ont les définitions indiquées dans la revendication 1,
lorsqu'on effectue la condensation intramoléculaire de composés de formule (II) 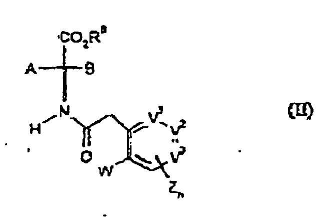 dans laquelle
A, B, W, V¹, V², V³, n et Z ont les définitions indiquées dans la revendication 1
et
R⁸ est un reste alkyle,
en présence d'un diluant et en présence d'une base,
puis, le cas échéant, on fait réagir les composés ainsi obtenus de formule (I-1-a)
(F)
α) avec des halogénures d'acides de formule (VIII) 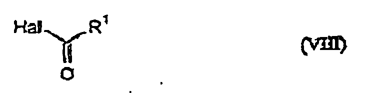 dans laquelle
R¹ a la définition indiquée dans la revendication 1 et Hal représente un halogène
ou bien
β) avec des anhydrides d'acide carbonylique de formule (IX)
R¹-CO-O-CO-R¹ (IX)
dans laquelle
R¹ a la définition indiquée ci-dessus,
le cas échéant en présence d'un diluant et en présence éventuelle d'un accepteur d'acide,
ou bien
(G) avec des esters d'acide chloroformique ou des thioesters d'acide chloroformique de formule (X)
R²-M-CO-Cl (X)
dans laquelle
R2 et M ont les définitions indiquées dans la revendication 1,
éventuellement en présence d'un diluant et, le cas échéant, en présence d'un accepteur d'acide,
ou bien
(H)
α) avec des esters d'acide chloromonothioformique ou des esters d'acide chlorodithioformique de formule (XI) 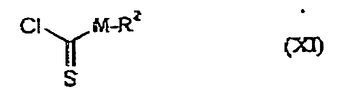 dans laquelle
M et R² ont les définitions indiquées ci-dessus,
éventuellement en présence d'un diluant et, le cas échéant, en présence d'un accepteur d'acide,
ou bien
β) avec du sulfure de carbone puis avec des composés de formule (XII)
R²-Hal (XIII)
dans laquelle
R² a la définition indiquée ci-dessus et
Hal représente le chlore, le brome ou l'iode,
éventuellement en présence d'un diluant et, le cas échéant, en présence d'une base,
ou bien
(I) avec des chlorures d'acide sulfonique de formule (XIII)
R³-SO₂-Cl (XIII)
dans laquelle
R³ a la définition indiquée dans la revendication 1,
éventuellement en présence d'un diluant et, le cas échéant, en présence d'un accepteur d'acide,
ou bien
(J) avec des composés de phosphore de formule (XIV) 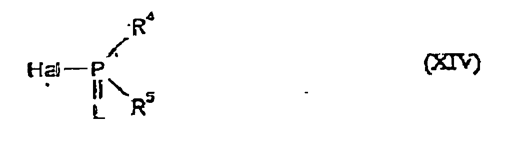 dans laquelle
L, R⁴ et R⁵ ont les définitions indiquées dans la revendication 1 et
Hal représente un halogène,
éventuellement en présence d'un diluant et, le cas échéant, en présence d'un accepteur d'acide,
ou bien
(K) avec des composés métalliques ou des amines de formule (XV) ou (XVI)
Me(OR¹⁰)ₜ (XV) 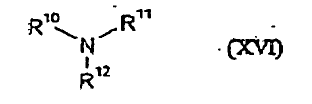
dans lesquelles
Me représente un métal monovalent ou divalent,
t représente le nombre 1 ou 2 et
R¹⁰, R¹¹, R¹² représentent, indépendamment les uns des autres, l'hydrogène ou un reste alkyle,
éventuellement en présence d'un diluant,
ou bien
(L)
α) avec des isocyanates ou des isothiocyanates de formule (XVII)
R⁶-N=C=L (XVII)
dans laquelle
R⁶ et L ont les définitions indiquées dans la revendication 1,
éventuellement en présence d'un diluant et, le cas échéant, en présence d'un catalyseur,
ou bien
β) avec des chlorures d'acide carbamique ou des chlorures d'acide thiocarbamique de formule (XVIII) 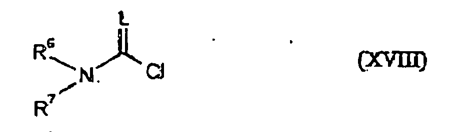 dans laquelle
L, R⁶ et R⁷ ont les définitions indiquées dans la revendication 1,
éventuellement en présence d'un diluant et, le cas échéant, en présence d'un accepteur d'acide.

3. Composés de formule (II) 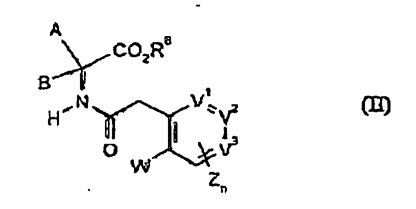 dans laquelle
W, X, V¹, V², V³ et n ont les définitions indiquées dans la revendication 1,
R⁸ est un reste alkyle, et
A représente un reste alkyle, alcényle, alkoxyalkyle, polyalkoxyalkyle ou alkylthioalkyle, chacun éventuellement substitué par un halogène, un reste cycloalkyle ou hétérocyclyle, chacun saturé ou non saturé et éventuellement substitué ou un reste aryle, arylalkyle ou hétaryle, chacun éventuellement substitué par un radical halogéno, alkyle, halogénalkyle, alkoxy, halogénalkoxy, cyano ou nitro,
B est un reste alkyle ou alkoxyalkyle, ou bien
A et B forment conjointement avec l'atome de carbone auquel ils sont liés un carbocycle saturé ou non saturé éventuellement substitué ou un hétérocycle.

4. Pesticides et herbicides **caractérisés par** une teneur en au moins un composé de formule (I) suivant la revendication 1.

5. Utilisation de composés de formule (I) suivant la revendication 1 pour combattre des parasites et des mauvaises herbes.

6. Procédé pour combattre des parasites et des mauvaises herbes, **caractérisé en ce qu'**on fait agir des composés de formule (I) suivant la revendication 1 sur des parasites ou des mauvaises herbes et/ou sur leur milieu.

7. Procédé de préparation de compositions pesticides et de compositions herbicides, **caractérisé en ce qu'**on mélange des composés de formule (I) suivant la revendication 1 avec des diluants et/ou des agents tensioactifs.
